# EUROPEAN PATENT APPLICATION

(11) **EP 1 958 648 A1**
(43) Date of publication of application: **20.08.2008**
(21) Application number: 06822468.2
(22) Date of filing: 27.10.2006
(51) Int. Cl.: A61K 48/00, A61K 35/12, A61K 35/14, A61K 35/76, A61K 38/43, A61P 7/04, C12N 5/00, C12N 15/00

(54) **THERAPEUTIC METHOD FOR BLOOD COAGULATION DISORDER**

(30) Priority: 28.10.2005 JP 2005315447
(71) Applicant: Dnavec Corporation, Tsukuba-shi, Ibaraki 305-0856 (JP)
(72) Inventor: OHMORI, Tsukasa, Shimotsuke-shi, Tochigi 3290431 (JP); SAKATA, Yoichi, Shimotsuke-shi, Tochigi 3290431 (JP); MITOMO, Katsuyuki, Tsukuba-shi, Ibaraki 3050856 (JP); TABATA, Toshiaki, Tsukuba-shi, Ibaraki 3050856 (JP); HASEGAWA, Mamoru, Tsukuba-shi, Ibaraki 3050856 (JP)
(74) Representative: Vossius & Partner
(86) International application number: PCT/JP2006/321507
(87) International publication number: WO 2007/049749

(57) **Abstract**

The present invention provides agents for treating blood coagulation abnormalities, which contain as an active ingredient a lentiviral vector carrying a blood coagulation factor gene operably linked to a promoter which induces platelet-specific expression. Agents for treating hemophilia A or hemophilia B are provided by application of the gene encoding Factor VIII or Factor IX. Blood coagulation abnormalities can be treated by gene therapy by infecting hematopoietic stem cells or such with the therapeutic agents of the present invention.

## Description

### Technical Field

The present invention relates to treatment of blood coagulation abnormalities.

### Background Art

Blood has a mechanism of stanching bleeding (hemostasis). Bleeding occurs due to vascular damage. Hemostasis is achieved mainly by: a process in which platelets gather at the damaged site of a blood vessel (primary hemostasis); and a process in which fibrin fills the space between platelets (secondary hemostasis). This series of processes to stop bleeding is referred to as blood coagulation. Blood coagulation is a complex physiological reaction in which many substances are involved in a complex manner. Primary hemostasis is a process of sealing damaged sites. Connective tissues that are exposed underneath damaged endothelial cells contain collagen and the like. Platelets adhere to these connective tissues via von Willebrand factor, and then form aggregates.

Meanwhile, the latter step which involves fibrin formation and accumulation is called secondary hemostasis. Fibrin is a fibrous protein formed by activating its precursor (fibrinogen) in the blood by the activity of proteases such as thrombin. The platelet aggregate structure formed by primary hemostasis serves as a scaffold for the formation of fibrin aggregates, and at the same time, the structure itself is strengthened by fibrin. To date, twelve types of substances, Factor I to Factor XIII (Factor VI is absent), have been identified as factors involved in fibrin formation, which plays a central role in secondary hemostasis. These substances are involved in the series of blood coagulation reactions, for example, by regulating the activities of other factors or by stabilizing fibrin.

Under normal conditions, the activities of many factors involved in blood coagulation are elaborately regulated in the blood. Thus, under normal conditions, blood that flows in the vessels does not coagulate. However, primary hemostasis begins immediately once bleeding occurs. Then, multiple factors that participate in secondary hemostasis are sequentially activated. Under normal conditions, this series of processes is a rapid physiological reaction that proceeds in a matter of seconds.

Abnormalities in the quantity or activity of the group of factors involved in blood coagulation lead to abnormality in the blood coagulation mechanism. For example, when the expression or activity of a factor supporting blood coagulation is reduced, the normal hemostasis system stops operating. Such condition is called blood coagulation abnormality. There are various known blood coagulation abnormalities that result from different causes. Hemophilia is a representative disease associated with blood coagulation abnormalities.

Hemophilia is classified into hemophilia A and hemophilia B according to the cause. The cause of hemophilia A is quantitative or qualitative abnormality of Factor VIII. Meanwhile, hemophilia B is caused by Factor IX deficiency. First, Factor VIII is a thrombin-activated blood coagulation factor. Activated Factor VIII (Factor VIIIa) binds to activated Factor IX (Factor IXa) and activates Factor X. Activated Factor X (Factor Xa) activates thrombin, thereby leading to fibrin formation. Meanwhile, Factor IX is activated by activated Factor XI (Factor XIa) and together with similarly activated Factor VIIIa, serves as an activation factor for Factor X. Both Factor VIII and Factor IX are important factors supporting the activation of thrombin, which is required at the final stage of secondary hemostasis. Thus, hemophilia, which is cause by deficiency of these factors, involves a critical blood coagulation failure depending on the severity.

In general, hemophilia is a genetic disease. Therefore, it is currently difficult to repair the causes of the disease, which are genetic causes. Thus, hemophilia is being treated by supplementing the deficient factors (substitution therapy). Factor VIII and IX purified from human blood have been used in substitution therapy. However, administration of blood formulations prepared from human blood resulted in damages such as infections by human immunodeficiency viruses and hepatitis viruses. Efforts are being made to reduce the risk of such infection by excluding infected blood, establishing techniques for inactivating pathogenic viruses, and so on. Furthermore, it is thought that the risk of infection caused by administration of blood coagulation factors has been almost eliminated by the application of purified protein formulations, which use factors produced by genetic engineering.

Even when the risk of infection is reduced, the current substitution therapy can constrain the patient's daily life. In the substitution therapy, in general, the dosage and schedule at which a blood coagulation factor is administered are set depending on the level of deficiency of the blood coagulation factor. Normally, blood coagulation factors retain the effect at the time of administration for about half a day after the administration; then, the effect decreases over time. For this reason, blood coagulation factors are administered every 6 to 24 hours. Protein formulations of blood coagulation factors are administered by intravenous injection. Such frequent intravenous injections can constrain the patient's daily life.

In addition, it is pointed out that substitution therapy for hemophilia has the problem of autoantibody production. Neutralizing antibodies (inhibitors) against administered blood coagulation factors are sometimes detected in hemophilia patients who receive substitution therapy. Antibodies produced by patients neutralize administered blood coagulation factors and thus inhibit their activity. As a result, a typical dosage does not produce sufficient therapeutic effect in patients that have autoantibodies.

For substitution therapy for hemophilia, gene therapy has been reportedly attempted as a method of administering blood coagulation factors that does not require intravenous injections. For example, Factor VIII was found to be expressed in platelets of transgenic mice into which a gene encoding Factor VIII is introduced, demonstrating the possibility of treating hemophilia by gene therapy (Blood (2003) 102: 4006-4013). However, when actually applying the therapy to human, expression of the exogenous Factor VIII gene must be induced by methods other than that for transgenic animals.

The use of viral vectors, rather than of transgenic animals, for inducing the expression of the Factor VIII gene has been attempted. In the case of viral vectors, they can be clinically applied to humans. Thus, lentiviral vectors into which a DNA encoding Factor VIII is incorporated were introduced into bone marrow cells and the like. Then, these bone marrow cells were transplanted into a mouse model of blood coagulation abnormality (Factor VIII-knockout mouse; Mol Ther. 2003, May; 7 (5 Pt 1):623-631). However, according to this report, although a strong expression of Factor VIII was seen *in vitro*, sufficient Factor VIII activity was not detected in the peripheral blood of the transplanted mouse. Induction of neutralizing antibodies against Factor VIII in the mouse was assumed to be the cause for the reduction in the *in vivo* activity of Factor VIII.

The present inventors introduced into CD34-positive cells of human cord blood a simian immunodeficiency virus vector incorporated with Factor VIII. Then, these cells were transplanted into NOD/SCID mice and the expression of Factor VIII in the blood was examined. As a result, the expression of the introduced Factor VIII was confirmed to last for at least 60 days (J Gene Med. 2004 Oct.; 6(10):1049-1060). However, since the NOD/SCID mice used in the experiment are in a severely immunodeficient condition, the effect of autoantibodies was not evaluated. Alternatively, the activity of platelets was enhanced by using the GPIIb promoter to express integrin, which is an adhesion factor in platelets, in a mouse model of Glanzmann thrombasthenia (GT) (Fang J. et al. Blood. 2005 Oct 15; 106(8):2671-9. Epub 2005 Jun 21.).
Non-Patent Document 1: Yarovoi HV. et al., Blood 2003; 102: 4006-4013.
Non-Patent Document 2: Kootstra NA. et al., Mol Ther. 2003, May; 7 (5 Pt 1):623-631.
Non-Patent Document 3: Kikuchi J. et al., J Gene Med. 2004 Oct.; 6(10):1049-1060.
Non-Patent Document 4: Fang J. et al., Blood. 2005 Oct 15; 106(8):2671-9. Epub 2005 Jun 21.

### Disclosure of the Invention

### [Problems to be Solved by the Invention]

An objective of the present invention is to provide techniques for treating blood coagulation abnormality by gene therapy. More specifically, an objective of the present invention is to achieve *in vivo* expression of genes encoding blood coagulation factors without relying on transgenic animal techniques or such, and to provide techniques for treating blood coagulation abnormalities by gene therapy.

### [Means for Solving the Problems]

To solve the aforementioned problems, the present inventors thought it would be effective if the expression of genes encoding blood coagulation factors could be induced in a platelet-specific manner. Blood coagulation factors expressed in platelets are retained within platelets and contact with the organism's immune system is prevented. Accordingly, the possibility of inducing neutralizing antibodies, which is problematic in conventional technology, is considered low. Meanwhile, platelets rapidly gather at sites of vessel damage upon bleeding. Platelets that are activated at the site of bleeding release various factors required for hemostasis to the outside of cells. Thus, blood coagulation factors that accumulate in the cells are expected to be released to the outside of the cells.

The present inventors thought that platelet-specific induction of the expression of blood coagulation factors would be an ideal gene expression system in gene therapy for blood coagulation abnormalities. Based on this thinking, the present inventors tried to establish a system for platelet-specific expression of blood coagulation factors. Then, the inventors successfully expressed blood coagulation factors in platelets by using promoters that enable platelet-specific gene expression.

Furthermore, application of lentiviral vectors was examined with the aim of treating blood coagulation abnormalities, which requires continuous provision of blood coagulation factors. Specifically, vectors to be used in gene therapy for blood coagulation abnormalities were constructed, and they are lentiviral vectors carrying a blood coagulation factor gene expressed under the control of a platelet-specific promoter. Then, the inventors confirmed that blood coagulation ability was actually improved in model animals into which cells introduced with this vector for gene therapy were transplanted, and thereby completed the present invention. Specifically, the present invention provides the following agents, methods, and kits for treating blood coagulation abnormalities.
[1] an agent for treating a blood coagulation abnormality, which comprises as an active ingredient a lentiviral vector comprising a promoter specific to megakaryocytes and/or platelets which are derivatives thereof, and a polynucleotide encoding a blood coagulation factor operably linked to the promoter;
[2] the therapeutic agent of [1], wherein the promoter is a GPIb promoter or a variant thereof;
[3] the therapeutic agent of [2], wherein the polynucleotide encoding a blood coagulation factor is linked to the promoter via 5' UTR;
[4] the therapeutic agent of [1], wherein the blood coagulation abnormality is hemophilia A and the blood coagulation factor is Factor VIII or a mutant thereof;
[5] the therapeutic agent of [1], wherein the blood coagulation abnormality is hemophilia B and the blood coagulation factor is Factor IX or a mutant thereof;
[6] the therapeutic agent of [1], wherein the blood coagulation abnormality is either hemophilia A or hemophilia B and the blood coagulation factor is Factor VII or a mutant thereof;
[7] the therapeutic agent of [1], wherein the lentiviral vector is a simian immunodeficiency virus vector;
[8] the therapeutic agent of [1], wherein the lentiviral vector is any one selected from the group consisting of equine infectious anemia virus vector, human immunodeficiency virus 1 vector, human immunodeficiency virus 2 vector, feline immunodeficiency virus vector, bovine febrile disease virus vector, and caprine arthritis encephalitis virus vector;
[9] a hematopoietic stem cell which has been infected with a lentiviral vector comprising a promoter specific to megakaryocytes and/or platelets which are derivatives thereof, and a polynucleotide encoding a blood coagulation factor operably linked to the promoter;
[10] a megakaryocyte and/or platelet which is a derivative thereof, infected with a lentiviral vector comprising a promoter specific to megakaryocytes and/or platelets which are derivatives thereof, and a polynucleotide encoding a blood coagulation factor operably linked to the promoter;
[11] a method for producing either or both of a megakaryocyte and a platelet in which a blood coagulation factor is accumulated, wherein the method comprises the steps of: infecting a hematopoietic stem cell with a lentiviral vector comprising a promoter specific to megakaryocytes and/or platelets which are derivatives thereof, and a polynucleotide encoding a blood coagulation factor operably linked to the promoter; and culturing the hematopoietic stem cell infected with the lentiviral vector until it differentiates into a group of cells comprising either or both of a megakaryocyte and a platelet which is a derivative thereof;
[12] a method for treating a blood coagulation abnormality, wherein the method comprises the steps of:
   (1) infecting a hematopoietic stem cell with a lentiviral vector comprising a promoter specific to megakaryocytes and/or platelets which are derivatives thereof, and a polynucleotide encoding a blood coagulation factor operably linked to the promoter; and
   (2) administering the hematopoietic stem cell of step (1) to a patient with blood coagulation abnormality;
[13] the method of [12], wherein the hematopoietic stem cell comprises a hematopoietic stem cell collected from a patient;
[14] the method of [13], wherein the hematopoietic stem cell comprises either or both of a bone marrow stem cell and a peripheral blood hematopoietic stem cell;
[15] the method of [12], wherein the hematopoietic stem cell of step (1) is cultured and then administered to the patient;
[16] a kit for treating blood coagulation abnormality, wherein the kit comprises the following elements:
   a: a lentiviral vector comprising a promoter specific to megakaryocytes and/or platelets which are derivatives thereof, and a polynucleotide encoding a blood coagulation factor operably linked to the promoter; and
   b: a reagent for collecting a hematopoietic stem cell;
[17] the kit of [16] for treating blood coagulation abnormality, wherein the kit additionally comprises the following element c:
   c: a culture medium for inducing the differentiation of a hematopoietic stem cell into a megakaryocyte;
[18] the kit of [17] for treating blood coagulation abnormality, wherein the culture medium for inducing the differentiation of a hematopoietic stem cell into a megakaryocyte comprises at least the following substances:
   transferrin;
   insulin;
   stem cell factor;
   thrombopoietin;
   interleukin-6;
   Flt-3 ligand; and
   soluble interleukin-6 receptor; and
[19] use of a lentiviral vector for producing an agent for treating blood coagulation abnormality, wherein the lentiviral vector comprises a promoter specific to megakaryocytes and/or platelets which are derivatives thereof, and a polynucleotide encoding a blood coagulation factor operably linked to the promoter.

The present invention also relates to the use of lentiviral vectors in the production of agents for treating blood coagulation abnormalities, wherein the lentiviral vectors comprise a promoter specific to megakaryocytes and/or platelets derived from megakaryocytes, and a polynucleotide encoding a blood coagulation factor operably linked to the promoter. The present invention also provides pharmaceutical packages for treating blood coagulation abnormalities, which comprise lentiviral vectors comprising a platelet-specific promoter and a polynucleotide encoding a blood coagulation factor operably linked to the promoter; and instructions indicating that the intended use is for the treatment of blood coagulation abnormalities.

### [Effects of the Invention]

Novel methods for treating blood coagulation abnormalities were actualized based on the present invention. By using agents of the present invention for treating blood coagulation abnormalities, blood coagulation factors can be expressed in platelets for a long period of time and the effect of improving blood coagulation ability is sustained. Since therapeutic effect can be expected for a long period of time, sufficient treatment can be achieved with a small number of treatments. Specifically, according to the present invention, blood coagulation ability can be stably maintained even in chronic blood coagulation abnormalities such as hemophilia with, for example, one treatment (administration) every few months.

The phenomenon that neutralizing antibodies are induced against blood coagulation factors administered for therapeutic purposes, thereby limiting the therapeutic effect is often observed in hemophilia patients. If therapeutic methods that can prevent the induction of neutralizing antibodies are put into practical use, they will be useful as therapeutic methods for hemophilia. In the present invention, blood coagulation factors that are expressed in platelets are retained within the cells. As a result, the chance that exogenous blood coagulation factors are in contact with the patient's immune system is markedly limited. Therefore, the present invention prevents the induction of neutralizing antibodies against blood coagulation factors administered for treatment.

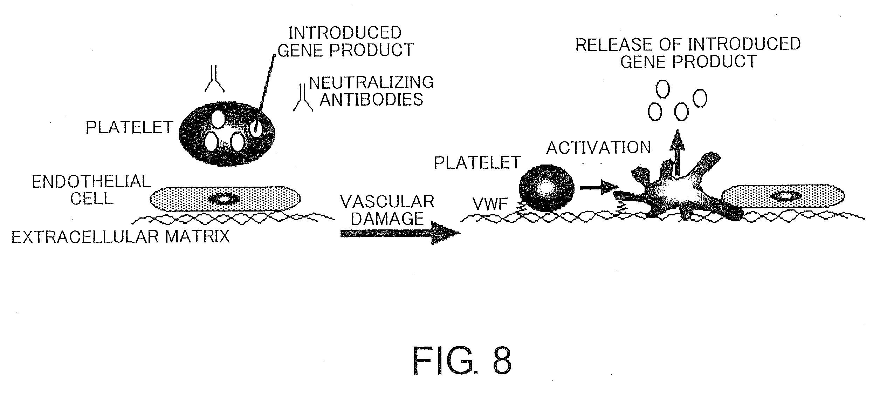

Furthermore, the blood coagulation abnormality therapy according to the present invention is also useful in treating hemophilia patients who already have neutralizing antibodies. In patients with neutralizing antibodies, blood coagulation factors administered in the blood bind to neutralizing antibodies and their activity is decreased. On the other hand, according to the present invention, the blood coagulation factors expressed in platelets do not contact neutralizing antibodies in the blood as long as the factors are retained within the platelets; therefore, their activity is maintained without being neutralized. Then, blood coagulation factors released from activated platelets at the bleeding site help blood coagulation at the bleeding site. The present invention's scheme for protection from neutralizing antibodies in the blood and release of blood coagulation factors at the bleeding site is shown in Fig. 8.

According to the present invention, the effect of preventing the induction of neutralizing antibodies against blood coagulation factors is particularly advantageous for blood coagulation factors secreted in the blood under normal conditions. In general, these humoral factors achieve therapeutic effect when they are retained in the blood. Therefore, contact of administered proteins with the patient's immune system is inevitable during treatment. Thus, prevention of the induction of neutralizing antibodies is a very difficult task. Meanwhile, in the present invention, sufficient therapeutic effect can be achieved by retaining the necessary blood coagulation factors within platelets even though they are humoral factors. In addition, blood coagulation factors retained in platelets can avoid contact with the immune system. Therefore, the present invention provides methods that solve both of the two difficult problems encountered when administering humoral blood coagulation factors: therapeutic effect and prevention of the induction of neutralizing antibodies.

### Brief Description of the Drawings

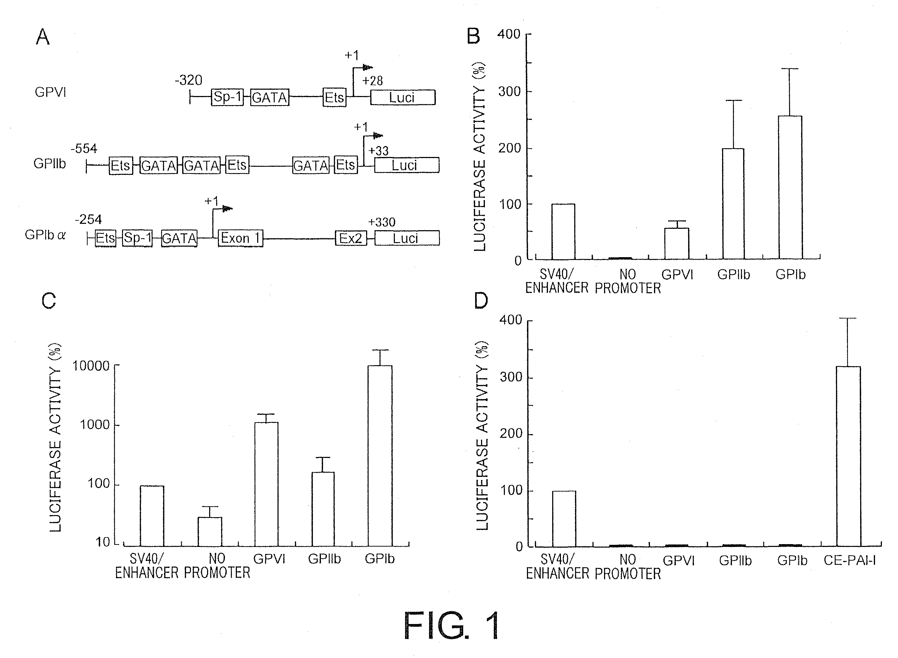

Fig. 1 shows a comparison of platelet-specific promoter activities. (A) shows a schematic presentation of platelet-specific promoters used in the experiments. Each construct was transfected, along with a promoter-less vector (basic) or a control vector (SV40/Enhancer), into UT-7/TPO (B), CD34⁺-derived megakaryocytes (C), and HUVEC (D). Luciferase activity was measured 48 hours after transfection, and is shown in relation to the activity promoted by the SV40 promoter (SV40/Enhancer). Each experiment was carried out five times with duplicate samples. Columns and error bars represent mean ± SD.

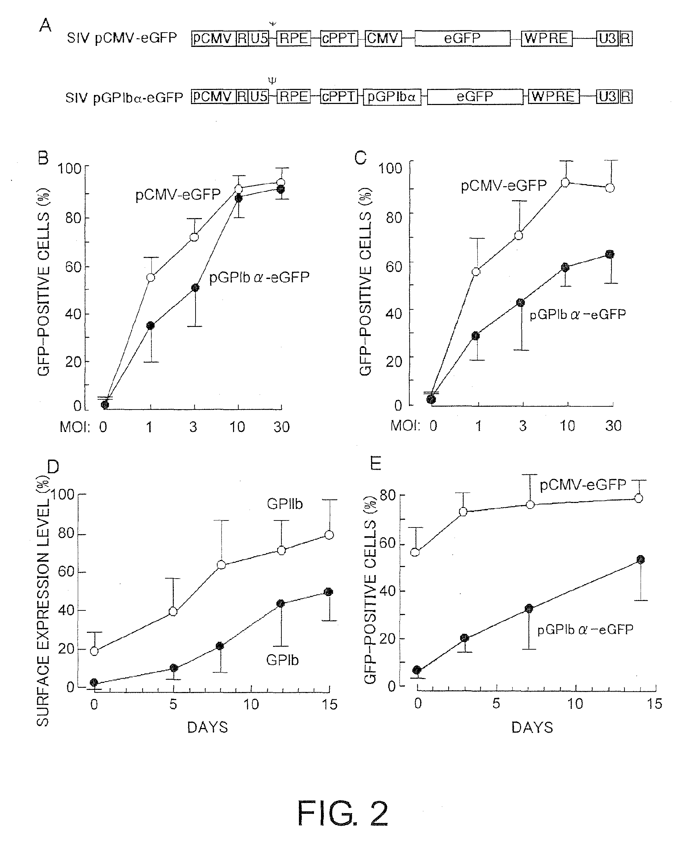

Fig. 2 shows the eGFP expression in UT-7/TPO cells and CD34⁺-derived megakaryocytes transduced with an SIV vector comprising the eGFP gene promoted by the CMV or GPIbα promoter. (A) shows a schematic diagram of SIV vectors used in this study. UT-7/TPO (B) and CD34⁺-derived megakaryocytes (C) are infected with SIV-pCMV-eGFP or SIV-pGPIbα-eGFP at indicated MOIs. The expression of eGFP in cells was analyzed by flow cytometry. Columns and error bars represent mean ± SD (n = 3). (D) The cell surface expression of GPIIb and GPIb during differentiation of human CD34⁺ cells into megakaryocytes was examined on Day 0, 5, 7, 12, and 15 by flow cytometry. Columns and error bars represent mean ± SD (n = 3). (E) CD34⁺-derived megakaryocytes were transduced with SIV-pCMV-eGFP or SIV-pGPIbα-eGFP at an MOI of 30 on Day 0, 3, 7, and 14 after the start of differentiation. Columns and error bars represent mean ± SD (n = 3).

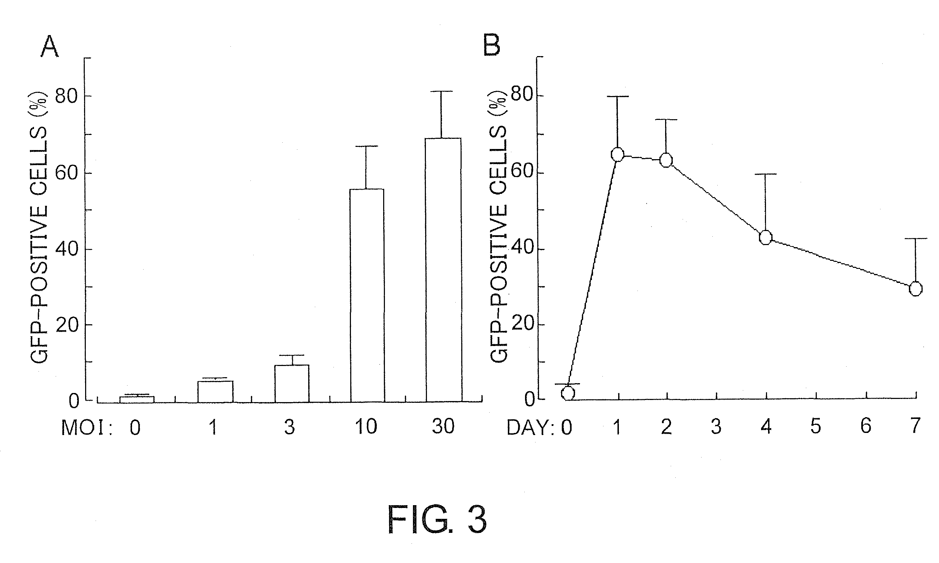

Fig. 3 shows the transduction of KSL cells by SIV-CMV-eGFP. Cultured KSL cells were transfected with increasing concentrations of SIV-pCMV-eGFP for 24 hours (A) or with a fixed concentration (MOI = 30) for various incubation times (B). After the specified incubation, the expression of eGFP in KSL cells was analyzed by flow cytometry. Percentages of transduced cells expressing eGFP are shown. Columns and error bars represent mean ± SD (n = 3).

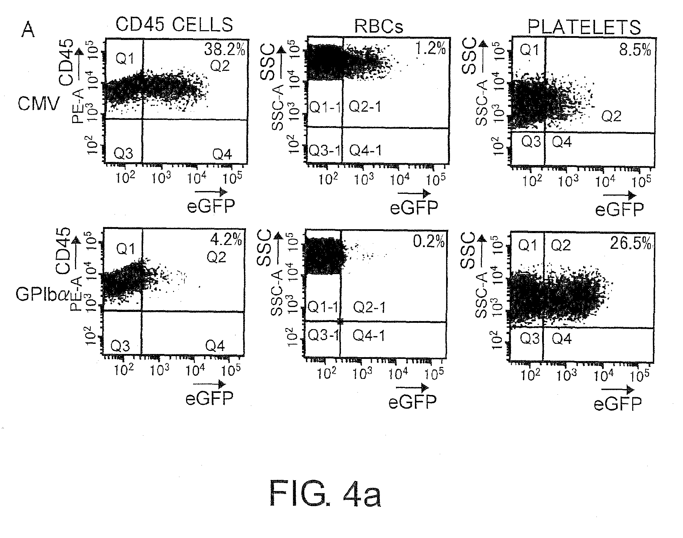

Fig. 4a shows the differential effects of promoters on eGFP expression in blood cells *in vivo*. Cultured KSL cells were transduced with SIV-pCMV-eGFP or SIV-pGPIbα-eGFP at an MOI of 30. Each irradiated mouse received 100,000 transduced cells together with 5 x 10⁵ unfractionated whole bone marrow cells. (A) shows representative flow cytometry analyses of eGFP-positive cells in CD45⁺ lymphocytes, and granulocytes, red blood cells (RBCs), and platelets in peripheral blood.

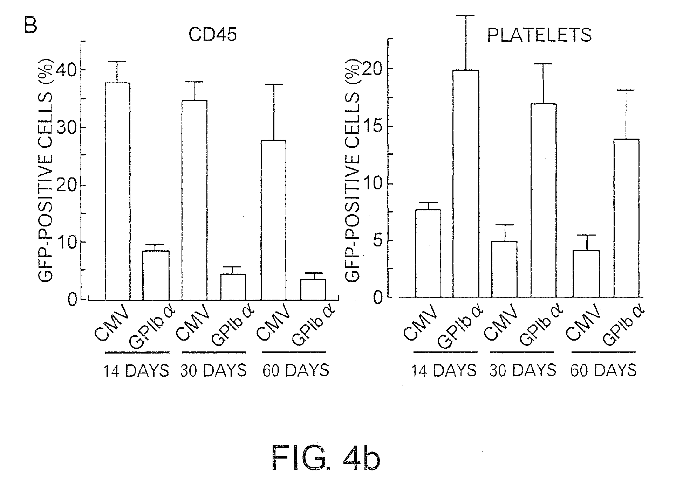

Fig. 4b (B) shows percentages of eGFP-positive cells in CD45⁺ lymphocytes (left) and platelets (right) on Day 14, 30, and 60 after transplantation. Columns and error bars represent mean ± SD (n = 5 in each group).

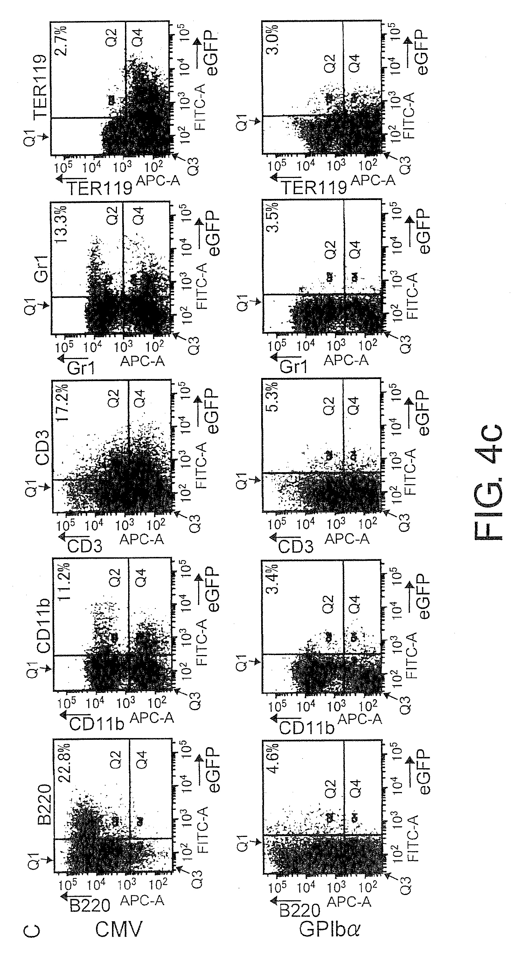

Fig. 4c (C) Bone marrow cells were stained using antibodies on post-transplantation day 60 to detect B lymphocytes (B220), T lymphocytes (CD3), granulocytes (Gr1), macrophages (CD11b), and erythroblasts (TER119). GFP-positive cells in the cells of each lineage were measured by flow cytometry. Data represent values from three experiments.

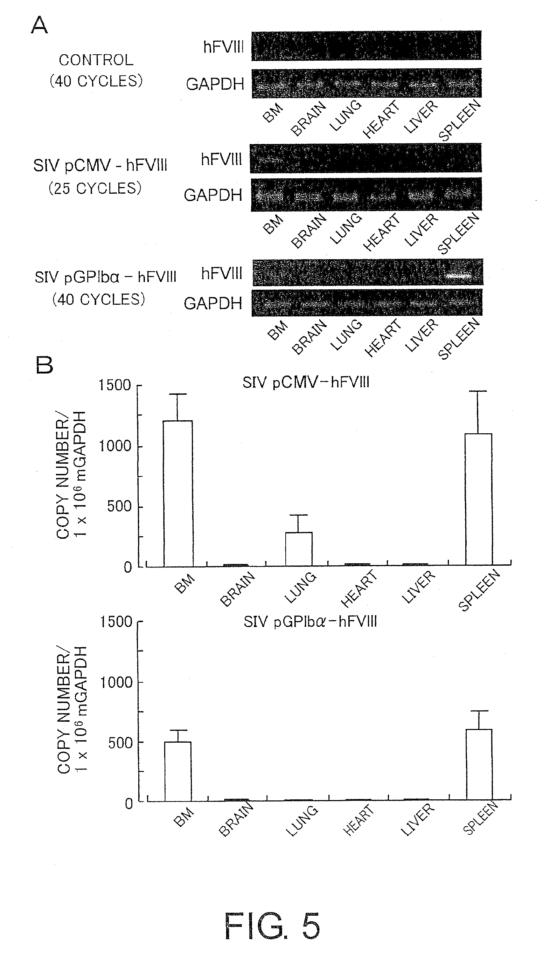

Fig. 5 shows the expression of hFVIII in organs obtained from mice transplanted with hFVIII-transduced KSL cells. (A) KSL cells not transduced (control) or transduced with SIV-pCMV-hFVIII or SIV-pGPIbα-hFVIII were injected into lethally irradiated CL57/B6 mice as described in the section "Materials and Methods". RT-PCR analyses of replicates derived from the hFVIII gene in the indicated organs are shown. At the same time, RT-PCR was performed on mouse GAPDH RNA, which serves as a control. Data represent central values from four experiments. (B) expression of the mRNAs derived from the SIV vectors was quantified by real-time RT-PCR, as described in the section "Materials and Methods". Columns and error bars represent mean ± SD (n = 4 in each group).

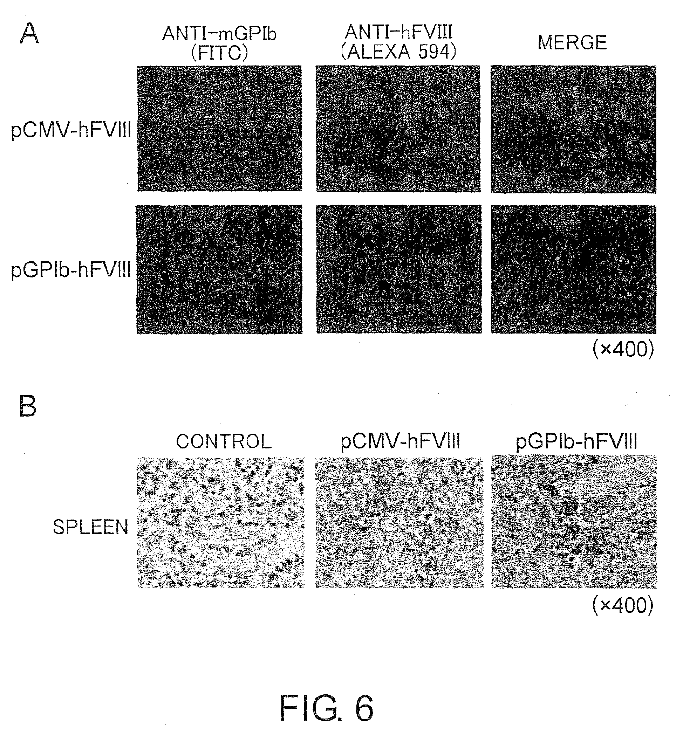

Fig. 6 shows hFVIII expression in the bone marrow and spleen of a transplanted mouse. (A) Bone marrow cells isolated from a mouse transplanted with KSL cells transduced with SIV-pCMV-hFVIII or SIV-pGPIbα-hFVIII were immunostained for mouse GPIbα (left) and hFVIII (middle). Two images are merged in the right column, showing sites of overlapping GPIbα and FVIII expressions in bone marrow cells transduced with SIV-pGPIbα-hFVIII. (B) Immunohistochemistry for hFVIII in the spleen of each transplanted mouse (positive stain: gray). As a control, spleen sections obtained from mice transplanted with KSL cells without vector infection were treated with an anti-FVIII antibody at the same time. Original magnification x 400.

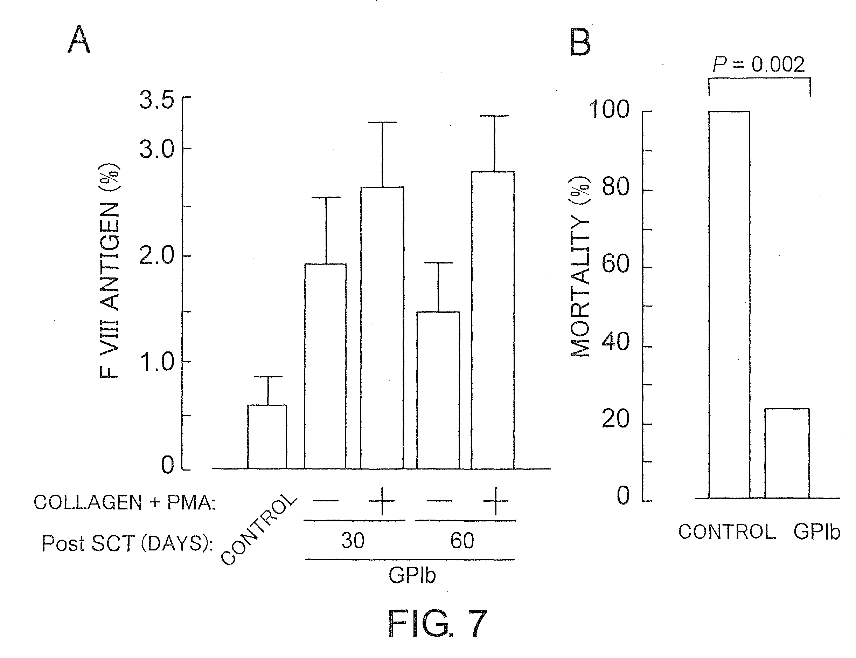

Fig. 7 shows phenotypic correction of hemophilia A mice by platelet-targeting gene delivery. (A) Blood from FVIII-deficient mice transplanted with KSL cells (transduced or not transduced with SIV-pGPIbα-hFVIII) is stimulated or not stimulated with 50 µg/ml collagen and 1 µM PMA for 15 minutes. After centrifugation, platelet-depleted plasma was obtained and the hFVIII antigen level was measured by ELISA. Columns and error bars represent mean ± SD (n = 4 in each group). (B) The mortality rate within 24 hours of cutting off the tails of mice transplanted with control KSL cells or SIV-pGPIbα-hFVIII-transduced KSL cells (n = 10 for the control; n = 8 for GPIbα). The mortality rate was statistically assessed by the chi-square test.
Fig. 8 schematically shows: protection of transduced gene products (blood coagulation factors) in platelets from neutralizing antibodies in the bloodstream; and release of the transduced gene products (blood coagulation factors) from activated platelets at a bleeding site.

### Best Mode for Carrying Out the Invention

The present invention relates to agents for treating blood coagulation abnormalities, which comprise, as an active ingredient, a lentiviral vector comprising a platelet-specific promoter and a polynucleotide encoding a blood coagulation factor operably linked to the promoter. The present inventors confirmed that the expression of a blood coagulation factor of interest can be induced in platelets by using a platelet-specific promoter. Furthermore, they confirmed that by introducing the gene into hematopoietic stem cells using a lentiviral vector, the blood coagulation factor is stably expressed for a long period of time, and blood coagulation ability is actually improved.

Herein, "blood coagulation factors" refers to proteins involved in blood coagulation. For example, Factor I to Factor XIII (Factor VI is absent) and their active forms are included in the blood coagulation factors of the present invention. Proteins involved in primary hemostasis, such as VWF, are also included in the blood coagulation factors of the present invention. The blood coagulation factors of the present invention are preferably derived from the same animal species as the subject to be treated. Thus, when the subject of the treatment is a human, blood coagulation factors derived from human are preferably used.

In the present invention, the blood coagulation factors include artificial proteins. Specifically, the proteins described below are included in the blood coagulation factors of the present invention.

### (1) Mutants having a similar activity as that of a natural blood coagulation factor

For example, human Factor VIII is known to retain activity as a blood coagulation factor even when it lacks the B domain at its N terminus (Blood Vol. 81, No.11, 1993: pp 2925-2935, Blood Vol. 84, No.12, 1994: pp 4214-4225, Eur J Biochem 1995 Aug.; 232(1): pp 19-27). Thus, Factor VIII mutants lacking the B domain are also included in the blood coagulation factors of the present invention. The amino acid sequence of human Factor VIII lacking the B domain is shown in SEQ ID NO: 10, and the nucleotide sequence of the coding region of the cDNA encoding the amino acid sequence is shown in SEQ ID NO: 9.

Similarly for other blood coagulation factors, mutants comprising one or multiple additions, deletions, substitutions, and insertions in their amino acid sequences and retaining a similar activity as natural blood coagulation factors are all included in the blood coagulation factors of the present invention.

Factor VIII is a large protein consisted of 2332 amino acids. The Factor VIII-encoding DNA extends to about 7 kb. Such a large protein cannot be sufficiently expressed by known vectors. Thus, to minimize the expression product, sometimes a B domain-deficient mutant (1457 amino acid residues) is used. However, the lentiviral vectors to be used for carrying the blood coagulation factor genes in the present invention have the characteristic that they can carry large foreign genes. Thus, they can carry not only mutants lacking the B domain but also, for example, a DNA encoding full-length Factor VIII, and maintain its stable expression.

Meanwhile, the blood coagulation factors of the present invention are preferably retained within platelets without being secreted after they are translated into proteins. When blood coagulation factors are secreted into the blood flow, they cause induction of neutralizing antibodies or neutralization by inhibitors. Thus, it is preferable that the blood coagulation factors of the present invention have a structure with no leader sequence.

### (2) Mutants having an activity stronger than that of natural blood coagulation factors

For the blood coagulation factors of the present invention, proteins having a similar activity as natural blood coagulation factors, as well as proteins having a modified property or a stronger activity than natural blood coagulation factors can be used. Blood coagulation factors that have a modified blood coagulation factor activity, modified stability *in vivo,* or modified immunogenicity toward the body can be obtained. Mutants containing mutations compared to natural blood coagulation factors and having an added therapeutically favorable trait can be used as blood coagulation factors of the present invention.

For example, Factor VIII variants (EXPERIMENTAL and MOLECULAR MEDICINE, Vol. 34, No. 3, 233-238, July 2002), Factor VII variants (Biochem. J. (2004) 379 (497-503)), and such have been reported. Such variants are comprised in the blood coagulation factors of the present invention.

In the present invention, for example, the blood coagulation factors shown below are useful for treating each of the diseases associated with blood coagulation abnormalities shown below. For each of the blood coagulation factors, mutants mentioned in (1) or (2) above can also be used as blood coagulation factors of the present invention.
Hemophilia A (Factor VIII and Factor VIIa)
Hemophilia B (Factor IX)
Von Willebrand's disease (von Willebrand factor)
Factor I deficiency, afibrinogenemia, and hypofibrinogenemia (Factor I)
Factor II deficiency (prothrombin)
Factor V deficiency and parahemophilia (Factor V)
Factor VII deficiency (Factor VII)
Factor X deficiency (Factor X)
Factor XI deficiency (Factor XI)
Factor XIII deficiency (Factor XIII)

Of these diseases, hemophilia A, hemophilia B, and von Willebrand's disease, which have a particularly large patient number, are socially important diseases. Thus, Factor VIII, Factor VIIa, Factor IX, and von Willebrand factor, which are useful in treating each of the diseases, and mutants having the same activity as these factors are preferred blood coagulation factors in the present invention.

Those skilled in the art can prepare polynucleotides encoding a blood coagulation factor of the present invention. For example, the amino acid sequences and the nucleotide sequences of cDNAs of human-derived VWF and Factor I to Factor XIII are known. Thus, polynucleotides of interest can be prepared from a liver cDNA library using the entire or a part of a cDNA nucleotide sequence as probe. Alternatively, polynucleotides of interest can be synthesized by known nucleic acid amplification methods using portions of a known nucleotide sequence as primers and mRNAs from appropriate tissues, such as liver, as template.

In the present invention, polynucleotides encoding a blood coagulation factor are operably linked to a platelet-specific promoter. Herein, "operably linked" means that a gene encoding a blood coagulation factor is linked downstream of a platelet-specific promoter so that the blood coagulation factor-encoding gene is transcribed according to the promoter activity. Alternatively, an operably linked promoter and gene means that the gene encoding a blood coagulation factor is expressed under the control of a platelet-specific promoter.

Meanwhile, herein, a "platelet-specific promoter" refers to a DNA that exhibits a promoter activity in platelets when introduced into arbitrary host cells. More specifically, for example, promoter activity can be detected in platelets or in megakaryocytes, which are platelet precursor cells, when a platelet-specific promoter is introduced into hematopoietic stem cells. Preferable promoters in the present invention are promoters with high transcriptional activity at the late stage of megakaryocyte differentiation. Meanwhile, when a promoter is not specific to platelets, promoter activity is detected in cells other than platelets and megakaryocytes.

The promoter activity in each cell can be assessed based on the principle of reporter assay. Specifically, a construct in which the reporter gene is operably linked to a promoter whose activity is to be assessed is prepared and introduced into various cells. The promoter activity is detected if a signal originating from the reporter gene is detected in cells introduced with the construct. Genes that generate various signals, such as fluorescence activity or enzymatic activity, are known as reporter genes. More specifically, GFP (fluorescent activity), LacZ or Luc (enzymatic activity), or such can be used as a reporter gene.

For the platelet-specific promoter activity in the present invention, among the group of cells into which the promoter is introduced, the promoter only needs to be specific to platelets. For example, when a promoter is introduced into hematopoietic stem cells or bone marrow cells, promoters whose platelet-specific promoter activity is positively confirmed in the group of introduced cells are included in the platelet-specific promoters of the present invention. There is no limitation on the type of platelet-specific promoter in the present invention. The promoter may originate from any animal species as long as it has the required activity. Alternatively, artificially modified promoters can also be used.

For example, the promoter of GPIbα, a platelet-specific membrane glycoprotein, is a suitable promoter for platelet- or megakaryocyte-specific expression of blood coagulation factor genes. Specifically, the activity of the GPIbα promoter is enhanced, in particular, at the late stage of megakaryocyte differentiation. In other words, the activity increases as the differentiation into platelet advances. Thus, the promoter is highly specific to platelets. Furthermore, the promoter activity is stronger than that of GPIIb, which is another platelet-specific promoter.

The nucleotide sequence of the GPIbα promoter which can be used in the present invention is shown in SEQ ID NO: 7. Furthermore, the transcriptional activity of a promoter comprising the nucleotide sequence of SEQ ID NO: 7 is increased by placing an untranslated region (5' UTR) between the promoter and the coding region of the gene expressed under the control of the promoter. Thus, for example, when the Factor VIII gene is operably linked to the promoter, the 5' UTR of the Factor VIII gene can be placed between GPIbα and the Factor VIII gene. A nucleotide sequence in which the 5' UTR of the Factor VIII gene is added downstream of GPIbα (SEQ ID NO: 7) is shown in SEQ ID NO: 8.

Herein, the GPIbα promoter includes promoters that are functionally equivalent to this promoter. Such functionally equivalent promoters can be found, for example, by homology search or the like based on the nucleotide sequence of SEQ ID NO: 7 (human) (for example, BLAST; Altschul, S. F. et al., 1990, J. Mol. Biol. 215: 403-410). Alternatively, they can also be obtained by genomic PCR using primers designed based on the nucleotide sequence of SEQ ID NO: 7. Alternatively, homologous promoters derived from non-human species can also be obtained by hybridization under stringent conditions, using a non-human animal genomic library and probes comprising the nucleotide sequence of SEQ ID NO: 7. Specifically, promoters of interest can be identified by preparing a probe from either a nucleic acid comprising the GPIbα promoter or a nucleic acid target of hybridization, and detecting hybridization of the probe with other nucleic acids.

Stringent hybridization conditions include, for example, conditions in which hybridization is carried out in a solution containing 5x SSC (1x SSC contains 150 mM NaCl and 15 mM sodium citrate), 7% (W/V) SDS, 100 µg/ml denatured salmon sperm DNA, and 5x Denhardt's solution (1x Denhardt's solution contains 0.2% polyvinylpyrrolidone, 0.2% bovine serum albumin, and 0.2% Ficoll) at 48°C, preferably at 50°C, and more preferably at 52°C; then washing is carried out with shaking for two hours at the same temperature as in hybridization, preferably at 60°C, more preferably at 65°C, and still more preferably at 68°C in 2x SSC, preferably in 1x SSC, more preferably in 0.5x SSC, and even more preferably in 0.1x SSC.

Promoters obtained as above comprise sequences that are highly homologous to the GPIbα promoter. A "highly homologous" sequence indicates a sequence exhibiting an identity of 70% or higher, preferably 75% or higher, more preferably 80% or higher, even more preferably 85% or higher, still more preferably 90% or higher, and yet more preferably 95% or higher. The sequence identity can be determined by, for example, using the BLAST program (Altschul, S. F. et al., 1990, J. Mol. Biol. 215: 403-410). Specifically, the blastn program is used to determine nucleotide sequence identity, while the blastp program is used to determine amino acid sequence identity. For example, calculation is carried out on the BLAST web page of NCBI (National Center for Biotechnology Information) by setting filters such as "Low complexity" to OFF and using default parameters (Altschul, S.F. et al. (1993) Nature Genet. 3:266-272; Madden, T.L. et al. (1996) Meth. Enzymol. 266:131-141; Altschul, S.F. et al. (1997) Nucleic Acids Res. 25:3389-3402; Zhang, J. & Madden, T.L. (1997) Genome Res. 7:649-656). For example, the following values may be used for the parameters.
open gap cost is set as 5 for nucleotide;
extend gap cost is set as 2 for nucleotide;
nucleotide mismatch penalty is set as -3;
reward for a nucleotide match is set as 1;
expect value is set as 10;
wordsize is set as 11 for nucleotide;
Dropoff (X) for blast extensions in bits is set as 20 in blastn; and
final X dropoff value for gapped alignment (in bits) is set as 50 in blastn.

The blast2sequences program (Tatiana A et al. (1999) FEMS Microbiol Lett. 174:247-250), which compares two sequences, can be used to prepare an alignment of two sequences and determine the identity of the sequences. Gaps are treated in the same way as mismatches and an identity value is calculated for the entire GPIbα promoter (for example, the entire sequence of SEQ ID NO: 7).

Furthermore, there can be polymorphic forms and variants of the GPIbα promoter. Moreover, in general, the promoter activity is often maintained even after partial substitution, deletion, or insertion in nucleotide sequence. Such GPIbα promoter variants can be used in the present invention. In general, GPIbα promoter variants can comprise a sequence with a substitution, deletion, and/or insertion of one or more nucleotides in the nucleotide sequence of SEQ ID NO: 7. In comparison with the nucleotide sequence of SEQ ID NO: 7, the difference is typically 30 nucleotides or less, preferably 20 nucleotides or less, more preferably 10 nucleotides or less, even more preferably 5 nucleotides or less, still more preferably 3 nucleotides or less, and yet more preferably 2 nucleotides or less. The promoter activity of the variants can be confirmed by, for example, methods such as the reporter assay described above. If the result confirms an activity similar to or above that of the GPIbα promoter, the promoter is a preferable GPIbα promoter of the present invention.

In the agents of the present invention for treating blood coagulation abnormalities, a polynucleotide encoding a blood coagulation factor operably linked to a platelet-specific promoter is incorporated into a lentiviral vector. Lentiviral vectors that can be used in the present invention are described below.

The life cycle of viruses can be divided mainly into an infection phase and growth phase. Generally, viral vectors are characterized in that they can utilize the viral infection system to efficiently introduce genes into host cells. To ensure safety, the self-replication ability of many viral vectors is eliminated by removing their growth system, thereby preventing them from growing in the introduced cells.

The structure of vector particles is briefly described below. First, vector particles have a protein outer shell called a capsid. The capsid is composed of structural proteins, which are gag gene products. A membrane structure called an envelope is present outside the capsid. The envelope has the function of determining the type of cell that can be infected. Two copies of vector genomic RNA, and a reverse transcriptase, a pol gene product, are present in the capsid. When viral vectors infect host cells, the vector genomic RNA is reverse transcribed by its own reverse transcriptase mentioned above, and then incorporated into the host chromosome to become a proviral DNA, thereby establishing the infection.

Generally, viral vectors can be prepared using packaging vectors and gene transfer vectors. Packaging vectors carry viral DNA in which the packaging signal has been removed. The viral DNA comprises viral protein sequences. Host cells into which packaging vectors have been introduced are particularly called packaging cells. When packaging vectors are introduced into hosts, due to the lack of a packaging signal, empty viral particles are formed in the host cells.

On the other hand, gene transfer vectors carry a foreign gene to be introduced and virus-derived gene sequences that are necessary for incorporation into host chromosomal DNA. In the present invention, a construct of a polynucleotide encoding a blood coagulation factor operably linked to a platelet-specific promoter is carried by the gene transfer vector as an exogenous gene. When such a gene transfer vector is introduced into packaging cells, vector genomic DNA provided by the gene transfer vector is integrated into the host chromosome, and then vector genomic RNA is produced by transcription. This vector genomic RNA is incorporated into viral particles produced by packaging cells, and viral particles capable of introducing nucleic acid molecules into hosts are produced.

Viral particles that lack the self-replication ability but have the ability to introduce nucleic acid molecules into hosts are generally called "viral vectors". In particular, viral vectors constructed by genetic recombination techniques are called "recombinant" viral vectors. Viral vectors constructed using packaging cells and DNAs encoding the viral genome are included in the "recombinant viral vectors". The lentiviral vectors in the present invention are viral particles that comprise a viral genome derived from lentivirus, lack the self-replication ability, and have the ability to introduce nucleic acid molecules into hosts.

"Lentivirus" refers to retroviruses belonging to the Lentivirus subfamily (Lentivirus). Lentivirus comprises, for example, the following viruses:
human immunodeficiency virus (HIV);
   (for example, HIV1 and HIV2);
simian immunodeficiency virus (SIV);
feline immunodeficiency virus (FIV);
maedi-visna virus;
equine infectious anemia virus (EIAV); and
caprine arthritis encephalitis virus (CAEV).

Lentiviral vectors derived from any strain or subtype can be used in the present invention. For example, HIV-1 includes all major (M) subtypes (including A to J), N, and outlier (O) (Hu, D. J. et al., JAMA 1996; 275: 210-216; Zhu, T. et al., Nature 1998, 5; 391(6667): 594-7; Simon, F. et al., Nat. Med. 1998, 4(9): 1032-7). Examples of isolated SIV strains include SIVagm, SIVcpz, SIVmac, SIVmnd, SIVsm, SIVsnm, and SIVsyk. Of these lentiviral vectors, simian immunodeficiency virus (SIV) vectors are particularly preferable viral vectors.

The genome nucleotide sequence of an SIV that can be used as an SIV vector in the present invention is shown in SEQ ID NO: 14. Another known SIV genome nucleotide sequence can also be used (Fukasawa et al. Nature, Vol. 333, p457-461, 1988; GenBank Accession No. X07805).

In the present invention, "simian immunodeficiency virus (SIV) vector" refers to a vector in which sequences (among the nucleic acid molecules in the viral particle) that are required for viral vector function are based on an SIV genome. In the present invention, the following regions (in order from the 5' side) can be shown as "sequences required for viral vector function":
R region in the 5' LTR;
U5 region;
packaging signal (φ);
RRE;
U3 region other than the promoter region in the 3' LTR; and
R region.

For example, the nucleotide sequences of each of the regions constituting the SIV vector of the present invention include the following nucleotide sequences:
SEQ ID NO: 1/ the nucleotide sequence from the 5' LTR region to the packaging signal;
SEQ ID NO: 2/ RRE sequence; and
SEQ ID NO: 3/ the nucleotide sequence from the U3 region to the R region lacking the promoter region of the 3'LTR.

These nucleotide sequences are only examples, and without being said, functionally equivalent nucleotide sequences can be used in the present invention. Thus, the SIV vectors of the present invention can comprise modifications as long as they fall within the above definition. For example, as long as the "sequences required for virus vector function" are derived from SIV, the vectors may comprise other SIV-derived sequences or non-SIV-derived sequences. Sequences that are preferably comprised include, for example, cPPT (central polypurine tract), CMV (an internal promoter), and WPRE (woodchuck hepatitis virus posttranscriptional regulatory element), and they will be discussed later.

Simian immunodeficiency viruses (SIVs) were discovered as HIV-like viruses in monkeys. SIVs constitute the primate lentivirus group together with HIVs (E. Ido and M. Hayami, "Genes, Infection and Pathogenicity of Simian Immunodeficiency Virus", Tanpakushitsu Kakusan Koso (Protein, Nucleic acid and Enzyme), Vo1.39, No.8, 1994). This group is further divided into the following four major subgroups:
1) the HIV-1 group, including HIV-1, which causes human acquired immune deficiency syndrome (AIDS), and SIVcpz, which was isolated from chimpanzees;
2) the HIV-2 group, including SIVsmm isolated from sooty mangabeys (*Cercocebus atys*), SIVmac isolated from rhesus monkeys (*Macaca mulatta*), and HIV-2, which shows pathogenicity in humans at low frequency (Jaffar, S. et al., J. Acquir. Immune Defic. Syndr. Hum. Retrovirol., 16 (5), 327-32, 1997);
3) the SIVagm group, represented by SIVagm isolated from African green monkeys (*Cercopithecus aethiops*); and
4) the SIVmnd group, represented by SIVmnd isolated from mandrills (*Papio sphinx*).

No pathogenicity in natural hosts has been reported for SIVagm and SIVmnd among those described above (Ohta, Y. et al., Int. J. Cancer, 15, 41(1), 115-22, 1988; Miura, T. et al., J. Med. Primatol., 18 (3-4), 255-9, 1989; M. Hayami, Nippon Rinsho, 47,1, 1989). In particular, the TYO-1 strain of the SIVagm virus, which was used in the Examples herein, has been reported to show no pathogenicity to natural hosts or to experimentally infected crab-eating monkeys (*Macaca facicularis*) and rhesus monkeys (*Macaca mulatta*) (Ali, M. et al., Gene Therapy, 1(6), 367-84, 1994; Honjo, S. et al., J. Med. Primatol., 19 (1), 9-20, 1990).

There are no reports of SIVagm infection and disease occurrence in humans, and its virulence against humans is not known. In general, primate lentiviruses have strict species-specificity, and there are few cases in which a virus was transmitted from a natural host to a different species and caused a disease. Alternatively, even when infection occurs, the disease tends to have a low incidence rate or to progress slowly (Novembre, F. J. et al., J. Virol., 71(5), 4086-91, 1997). Accordingly, viral vectors that are produced based on SIVagm, in particular, the SIVagm TYO-1 strain, are considered to be safer than vectors based on HIV-1 or other lentiviruses. Thus, lentiviral vectors produced based on the SIVagm TYO-1 strain are preferable in the present invention. The genomic nucleotide sequence of the SIVagm TYO-1 strain is shown in SEQ ID NO: 14.

The simian immunodeficiency virus vectors of the present invention can comprise a portion of a genomic RNA sequence of another retrovirus. The SIV vectors of the present invention also include, for example, vectors comprising a chimeric sequence in which part of the SIV genome has been substituted with a portion of the genomic sequence of a non-SIV lentivirus, such as:
human immunodeficiency virus (HIV);
feline immunodeficiency virus (FIV) (Poeschla, E. M. et al., Nature Medicine, 4(3), 354-7, 1998); and
caprine arthritis encephalitis virus (CAEV) (Mselli-Lakhal, L. et al., Arch. Virol., 143(4), 681-95, 1998)

The lentiviral vectors of the present invention include recombinant SIV vectors carrying a polynucleotide encoding a blood coagulation factor which is operably linked to a platelet-specific promoter. When the blood coagulation factor used in the present invention is Factor VIII, the SIV vector carrying a Factor VIII-encoding polynucleotide is referred to as SIV-FVIII vector. There is no limitation on the type or structure of the SIV-FVIII vector of the present invention as long as it falls within the above definition. Preferred SIV-FVIII vectors of the present invention include SIV vectors produced by using a gene transfer vector comprising a nucleotide sequence into which a polynucleotide encoding Factor VIII is inserted.

The SIV-FVIII vector of the present invention may be pseudotyped with VSV-G. The term "pseudotyping with VSV-G" refers to incorporating the VSV-G protein, a surface glycoprotein of vesicular stomatitis virus (VSV), into the envelope of the vector. The VSV-G protein may be derived from an arbitrary VSV strain. Examples of VSV-G proteins include, but are not limited to, proteins derived from the Indiana serotype strain (J. Virology 39: 519-528 (1981)). Alternatively, the VSV-G protein can be a modified VSV-G protein derived from the original protein by, for example, substituting, deleting, and/or adding one or more amino acids. VSV-G-pseudotyped vectors can be prepared by allowing the VSV-G protein to be present during viral production.

Viral particles produced in packaging cells can be pseudotyped with VSV-G by expressing VSV-G in these cells. This can be facilitated by, for example, transfection of a VSV-G expression vector, or induced expression of the VSV-G gene integrated into the host's chromosomal DNA. Since VSV-G protein is present on the membrane as a stable glycoprotein homotrimer, vector particles suffer little deterioration during purification. Moreover, virus particles pseudotyped with VSV-G can be concentrated to high concentrations using centrifugation (Yang, Y. et al., Hum Gene Ther: Sep, 6(9), 1203-13. 1995).

The SIV-FVIII vector of the present invention can additionally comprise envelope proteins derived from other viruses. For envelope proteins that can be added to the SIV-FVIII vector, for example, an envelope protein derived from a virus that infects human cells is preferred. Such envelope proteins are not particularly limited. For example, retroviral amphotropic envelope proteins can be added to the SIV-FVIII vector.

Specifically, for example, the envelope protein derived from the murine leukemia virus (MuLV) 4070A strain can be used as a retroviral amphotropic envelope protein. Alternatively, the envelope protein derived from MuMLV 10A1 can also be used (for example, pCL-10A1 (Imgenex) (Naviaux, R. K. et al., J. Virol. 70: 5701-5705 (1996)). Also included are proteins from the herpes virus family, such as the gB, gD, gH, and gp85 proteins from the herpes simplex virus, and the gp350 and gp220 proteins from the EB virus. Proteins from the Hepadna virus family may include the S protein of hepatitis B virus.

In the recombinant simian immunodeficiency virus vector of the present invention, the LTR (long terminal repeat) may also be modified. The LTR is a retrovirus-specific sequence, which is present at both ends of the viral genome. The 5' LTR serves as a promoter, enhancing proviral mRNA transcription. Thus, it is possible to enhance mRNA transcription of the gene transfer vector and to improve packaging efficiency and vector titer if the portion exhibiting the 5' LTR promoter activity in the gene transfer vector that encodes viral RNA genome packaged into viral particles, is substituted with another promoter having stronger promoter activity.

Furthermore, for example, in the case of lentiviruses, viral tat protein is known to enhance 5' LTR transcription activity, and therefore, substitution of the 5' LTR for a promoter not dependent on the tat protein enables the exclusion of tat from the packaging vector.

The RNA of viruses which have infected and invaded cells is reverse transcribed, and the subsequent linking of the LTRs at both ends forms a circular structure. Then, viral integrase couples with the linkage site between LTRs, and the viral genome is then integrated into cell chromosomes.

The transcribed proviral mRNA is the region ranging from the 5' LTR transcription initiation site to the 3' LTR polyA sequence located downstream. The 5' LTR promoter portion is not packaged in the virus. Thus, even if the promoter is replaced with another sequence, the portion inserted into target cell chromosomes is unchanged. Given the facts as described above, it is proposed that substitution of the 5' LTR promoter will yield a safer vector with a higher titer. Thus, substitution of the 5' end promoter of a gene transfer vector can increase the titer of a packageable vector.

Safety can also be increased by preventing transcription of the full-length vector mRNA in target cells. This is achieved using a self-inactivating vector (SIN vector) prepared by partially eliminating the 3' LTR sequence. The lentivirus provirus invading the target cell chromosomes has its 5' end bound to the U3 portion of its 3' LTR. Thus, following reverse-transcription, transcripts of the gene transfer vector are integrated into target cell chromosomes such that the U3 portion is at the 5' end. From this point begins the transcription of RNA with a structure similar to the gene transfer vector transcripts.

If there were lentivirus or related proteins in target cells, it is possible that the transcribed RNA would be re-packaged and infect other cells. There is also a possibility that the 3' LTR promoter might express host genes located adjacent to the 3' end of the viral genome (Rosenberg, N., Jolicoeur, P., Retroviral Pathogenesis. Retroviruses. Cold Spring Harbor Laboratory Press, 475-585, 1997). These are already considered to be problems of retroviral vectors, and the SIN vector was developed as a way of overcoming these problems (Yu, S. F. et al., Proc. Natl. Acad. Sci. U S A, 83(10), 3194-8, 1986).

When the 3' LTR U3 portion is deleted from a gene transfer vector, target cells lack the promoters of 5' LTR and 3' LTR, preventing the transcription of the full-length viral RNA and host gene. Furthermore, since only the genes of interest are transcribed from endogenous promoters, highly safe vectors capable of high expression can be expected. Such vectors are preferred in the present invention. SIN vectors can be constructed according to methods known in the art, or methods as described in Examples 1 to 4 of WO 2002/101057, which is a patent application by the present inventors.

One problem encountered in gene therapy using viral vectors that have the LTR sequence in the genome, (including retroviral vectors) is a gradual decrease in expression of the introduced gene. One factor behind this may be that when such a vector is integrated into the host genome, a host mechanism methylates the LTR, suppressing expression of the introduced gene (Challita, P. M. and Kohn, D. B., Proc. Natl. Acad. Sci. USA 91:2567, 1994). One advantage of SIN vectors is that reduction of gene expression by methylation of the LTR hardly occurs, because the vector loses most of the LTR sequence upon integration into the host genome.

The present inventors revealed that an SIN vector, prepared by substituting another promoter sequence for the 3' LTR U3 region of the gene transfer vector, maintained a stable expression for more than two months after introduction into primate ES cells (WO 2002/101057). Thus, an SIN vector designed to self-inactivate by the modification of the LTR U3 region is particularly suitable in the present invention.

Specifically, the present invention includes modified vectors in which one or more nucleotides in the 3' LTR U3 region have been substituted, deleted, and/or added. The U3 region may simply be deleted, or another promoter may be inserted into this region. Such promoters include, for example, the CMV promoter, the EF1 promoter, and the CAG promoter.

Furthermore, blood coagulation factor genes carried by the lentiviral vectors of the present invention are designed to be transcribed by a platelet-specific promoter. For example, when the LTR U3 region is, as described above, replaced with a platelet-specific promoter, which is a non-LTR promoter, the blood coagulation factor genes can be driven by this modified LTR. Alternatively, as shown in the Examples, the expression of a blood coagulation factor gene can be induced independently of LTR by placing a platelet-specific promoter within the LTR region, and linking the blood coagulation factor gene downstream of this position.

Regarding lentivirus vectors, such as the HIV vector, it has been pointed out when the host genome already carries an HIV provirus, there is a risk that recombination occurs between the exogenous vector and the endogenous provirus, and replicable viruses may be produced. This will indeed pose a serious problem in the future when HIV vectors are used in HIV patients. The SIV vector, however, has almost no sequence homology to HIV. In addition, the SIV vector is a replication-incompetent virus in which 80% or more of the virus-derived sequence has been removed. Thus, the risk that replicable viruses are produced is very low. Accordingly, they are safer than other lentivirus vectors.

The SIV vector of the present invention is a vector in which a certain percentage or more of the SIV genomic sequence has been removed, except for the above-described "sequences necessary for functioning as a virus vector". The preferred SIV vectors of the present invention are replication-incompetent viruses in which 40% or more, usually 50% or more, more preferably 60% or more, even more preferably 70% or more, and still more preferably 80% or more of the genomic sequence of the SIV from which the vectors are derived has been removed.

To produce retroviruses, a gene transfer vector DNA comprising a packaging signal is transcribed in host cells and virus particles are formed in the presence of the gag, pol and envelope proteins. The gag and pol proteins in the packaging cells can be provided using packaging vectors. The envelope proteins can be provided by packaging vectors or other vectors. For example, they can be supplied using a VSV-G expression vector.

A gene transfer vector of the present invention comprises at least the following elements:
5' LTR;
packaging signal sequence;
platelet-specific promoter;
blood coagulation factor gene; and
3' LTR sequence.

The LTR sequences can be modified as described above as modifications of the SIV vector. The above-described cPPT sequence, CMV sequence, RRE sequence, or such can be incorporated as well. The packaging signal sequence encoded by the gene transfer vector DNA should preferably be sufficient in length to maintain the structure formed by the sequence.

However, in order to suppress the frequency of wild-type virus formation, which occurs due to recombination of the vector DNA packaging signal and the packaging vector supplying the gag and pol proteins, it is also necessary to keep sequence overlapping between these vector sequences to a minimum. Therefore, when it comes to the construction of the gene transfer vector DNA, it is preferable to use a sequence which is as short as possible and yet still contains the sequence essential for packaging, to ensure packaging efficiency and safety.

For example, when the packaging vector is derived from SIVagm, the HIV-derived gene transfer vector is not packaged. Thus, the packaging signal to be used in the gene transfer vector DNA is considered to be limited to SIV origin. However, the SIV-derived gene transfer vector is also packaged when an HIV-derived packaging vector is used. Thus, the frequency of recombinant virus formation can be reduced if the vector particles are formed by combining a gene transfer vector and packaging vector derived from a different lentivirus. SIV vectors thus produced are also included in vectors of the present invention. In such cases, it is preferable to use combinations of primate lentiviruses (for example, HIV and SIV).

In a preferred gene transfer vector DNA, the gag protein is modified such that it is not expressed. Viral gag protein may be detected by a living body as a foreign substance, and thus may show antigenicity. Alternatively, the protein may affect cellular functions. To prevent gag protein expression, nucleotides downstream of the gag start codon can be added, deleted, or such, to introduce frameshift-causing modifications. It is also preferable to delete portions of the coding region of the gag protein.

The 5' side of the coding region of the gag protein is known to be essential for virus packaging. Thus, in a gene transfer vector, it is preferable that the coding region for the gag protein is deleted at the C terminus. It is preferable to delete as large a portion of the gag coding region as possible, so long as the deletion does not considerably affect the packaging efficiency. It is also preferable to replace the start codon (ATG) of the gag protein with a codon other than ATG. The replacement codon can be selected appropriately so as not to greatly affect the packaging efficiency. A viral vector can be produced by introducing the constructed gene transfer vector DNA, which includes the packaging signal, into appropriate packaging cells. The viral vectors produced can be recovered from, for example, the culture supernatant of packaging cells.

Furthermore, a gene transfer vector DNA can be modified to increase the transfer and expression efficiency of the blood coagulation factor gene. For example, the cPPT sequence can be introduced to increase transfer efficiency. cPPT is a sequence originally present in the SIV genome. cPPT has been reported for HIV viruses since quite some time ago (P. Charneau et al.: J. Virol. 65: 2415-2431, 1991). It has been reported that cPPT introduced in HIV vectors improves the transfer of the vector genome to nuclei and increases the gene transfer efficiency (A. Sirven et al.: Blood 96:4103-4110, 2000).

The nucleotide sequence of cPPT used in the Examples is shown in SEQ ID NO: 4. Meanwhile, an example of a modification that increases the expression efficiency is introduction of a WPRE sequence. WPRE is a factor that has a function of increasing gene expression efficiency (US Patent 6,284,469: RNA export element and methods of use). In other lentiviral vectors, simultaneous introduction of the two factors, cPPT and WPRE, has been reported to further enhance the effects of each factor (SC. Barry et al.: Hum. Gene Ther. 12: 1103-1108, 2001). The nucleotide sequence of WPRE used in the Examples is shown in SEQ ID NO: 5.

In the SIV-FVIII vectors of the present invention, cPPT can be placed at the same position as in ordinary lentiviral vectors. For example, cPPT may be placed between the promoter and the exogenous gene, or placed upstream of the RRE sequence; however, it is preferably placed upstream of the above-described platelet-specific promoter, which drives the transcription of the blood coagulation factor (Fig. 2A). Meanwhile, WPRE can be positioned downstream of the blood coagulation factor gene.

In the packaging vectors of the present invention, sequences that are unnecessary for the introduction of the blood coagulation factor genes can be removed. Examples of such unnecessary sequences include vif and vpr, which are called accessory genes, and the regulatory genes tat and rev. Accessory gene products have been reported to be not essential in vectors (V. Kim et al.: J. Virol 72: 811-816, 1998), and therefore accessory gene-deleted vectors have been recently used to improve safety. Furthermore, even safer vectors called third generation vectors have been developed by deleting tat and transferring rev to a different plasmid. When rev is removed from the packaging vector, a rev expression vector can be constructed separately and used to produce SIV-FVIII vectors of the present invention. The nucleotide sequence of rev of the SIVagm TYO-1 strain is shown in SEQ ID NO: 6.

Packaging vectors constructed as described above can comprise, for example, a promoter sequence, a virus core protein sequence (gag), a reverse transcriptase sequence (pol), and a polyA sequence. The packaging vector can further comprise an RRE sequence as well as the above components, as indicated in the Examples below. In addition, the rev expression vector may be constructed such that a promoter for regulating the rev sequence is positioned upstream of the rev sequence, and a polyA sequence is positioned downstream of the rev sequence.

There is no limitation on the type of packaging cell, so long as the cell line is generally used in viral production. When used for human gene therapy, a human- or monkey-derived cell is suitable. Human cell lines that can be used as packaging cells include, for example, 293 cells, 293T cells, 293EBNA cells, SW480 cells, u87MG cells, HOS cells, C8166 cells, MT-4 cells, Molt-4 cells, HeLa cells, HT1080 cells, TE671 cells, etc. Monkey-derived cell lines include, for example, COS1 cells, COS7 cells, CV-1 cells, BMT10 cells, etc.

The SIV-FVIII vectors of the present invention can be substantially purified. Specifically, the vectors can be purified using known purification and separation methods, such as filtration, centrifugation, and column purification. For example, a vector can be precipitated and concentrated by filtering a vector solution with a 0.45-µm filter, and then centrifuging it at 42500 x g at 4°C for 90 minutes.

Agents of the present invention for treating blood coagulation abnormalities can be used to treat or prevent diseases associated with blood coagulation abnormalities. Specifically, the agents can be used to treat or prevent hemophilia. For example, when Factor VIII or Factor VIIa (activated Factor VII) is used as a blood coagulation factor, the agent can be used as therapeutic agent for hemophilia A. Alternatively, the agent can be used as a therapeutic agent for hemophilia B by using Factor IX as a blood coagulation factor. The above-described SIV vectors carrying a blood coagulation factor gene operably linked to a platelet-specific promoter can be appropriately combined with a desired pharmaceutically acceptable carrier or vehicle, as required, to prepare the agents for treating the diseases described above.

The term "pharmaceutically acceptable carrier" refers to a material that can be administered together with the vector and which does not significantly inhibit gene transfer mediated by the vector. Specifically, the vector can be appropriately combined with, for example, sterilized water, physiological saline, medium, serum, and phosphate buffered saline (PBS). In addition, a stabilizer, biocide, and such can also be included.

Platelet precursor cells can be infected with the above-described agents for treating blood coagulation abnormalities to produce platelet precursor cell compositions for transplantation. Alternatively, megakaryocytes or platelets for treating blood coagulation abnormalities can also be produced by differentiating these cells into megakaryocytes or platelets. Thus, the present invention relates to platelet precursor cells infected with a lentiviral vector comprising a platelet-specific promoter and a polynucleotide encoding a blood coagulation factor operably linked to the promoter.

In the present invention, the platelet precursor cells include any cells that have the ability to differentiate into platelets. For example, hematopoietic stem cells are cells that have the ability to differentiate into platelets. Alternatively, megakaryocytes differentiated from hematopoietic stem cells are cells that further differentiate into platelets. Therefore, megakaryocytes are also platelet precursor cells.

The present invention relates to methods for producing either or both of megakaryocytes and platelets that have accumulated a blood coagulation factor, which comprise the steps of: infecting platelet precursor cells with a lentiviral vector comprising a platelet-specific promoter and a polynucleotide encoding a blood coagulation factor operably linked to the promoter; and culturing the platelet precursor cells infected with the lentiviral vector until they differentiate into a group of cells comprising either or both of megakaryocytes and platelets.

For example, differentiation of hematopoietic stem cells into megakaryocytes is induced by culturing stem cells in the presence of appropriate cytokines or such. If culture is continued, megakaryocytes which are platelet precursor cells further differentiate to platelets. The differentiation of hematopoietic stem cells into megakaryocytes or platelets can be detected by verifying the disappearance or emergence of markers specific to each cell. Alternatively, differentiation into megakaryocytes or platelets can be confirmed by observing the change in cell morphology. Megakaryocytes and platelets have characteristic cell morphologies, and thus their morphological changes can be easily recognized.

In the present invention, hematopoietic stem cells (HSCs) refers to cells that can differentiate into at least either blood cell: megakaryocytes or platelets. In other words, all platelet precursor cells that differentiate into platelets are included in the hematopoietic stem cells of the present invention. In the present invention, cell groups comprising these cells can be used as the hematopoietic stem cells. Specifically, for example, bone marrow and cord blood can be used as a cell group comprising hematopoietic stem cells. Alternatively, peripheral blood stem cells can also be used as the hematopoietic stem cells.

Each blood cell can be identified based on its morphology or staining specificity. Staining methods that can be used include Giemsa staining, Wright's stain, May-Giemsa staining, May-Grunwald-Giemsa staining, peroxidase staining, elastase staining, and PAS staining (see Shin seikagaku j ikken kouza 8 (New Courses in Experimental Biochemistry 8), Ketsueki (Blood) Vol. 1, 1987, p. 8-15).

Hematopoietic stem cells are present not only in the bone marrow, but also in the peripheral blood and cord blood. The present invention's therapy for blood coagulation abnormalities is performed by infecting these cells with the lentiviral vector of the present invention and administering these to patients. In the present invention, the administration of hematopoietic stem cells refers to administration of cellular compositions comprising hematopoietic stem cells to patients. Specifically, these include bone marrow transplantation (BMT), peripheral blood stem cell transplantation (PBSCT), and cord blood stem cell transplantation (CBSCT). Thus, the present invention is applicable not only to bone marrow cell transplantation, but also to peripheral blood hematopoietic stem cell transplantation and cord blood transplantation. HSC can be identified by counting precursor cells of granulocytes and monocytes using colony assay, or by determining cells positive for CD34 (antigen), which is believed to be characteristic of HSC, using a flow cytometer.

Specifically, hematopoietic cells include cells exhibiting c-kit⁺, Thy 1.1^{low}, lin⁻, and Sca-1⁺ phenotype for mice (Osawa M, Hanada K, Hamada H, Nakauchi H. Science. 1996; 273: 242-245.), and cells exhibiting CD34⁺, Thy⁺, Lin⁻, and c-Kit^{low} phenotype for humans (Bhatia M, Wang JC, Kapp U, Bonnet D, Dick JE. Proc Natl Acad Sci USA. 1997; 94:5320-5325). The lin⁻ (lineage negative) phenotype can be identified and selected using commercially available kits or the like. Such cells are negative, for example, for all of GPA, CD3, CD2, CD56, CD24, CD 19, CD14, CD16, and CD99b.

Methods for separating human hematopoietic stem cells can also be referred to in the following documents:
Leary AG, Blood 69: 953, 1987;
Sutherland HJ, Blood 74: 1563, 1989;
Andrews RG, J Exp Med 169: 1721. 1989;
Terstappen LWMM, Blood 77: 1218, 1991;
Lansdorp PM, J Exp Med 175: 1501, 1992;
Briddell RA, Blood 79: 3159, 1992;
Gunji Y, Blood 80: 429, 1992;
Craig W, J Exp Med 177: 1331, 1993;
Gunji Y, Blood 82: 3283, 1993;
Traycoff CN, Exp Hematol 22: 215,1994;
Huang S, Blood 83: 1515, 1994;
DiGinsto D, Blood 84: 421, 1994;
Murray L, Blood 85: 368, 1995;
Hao QL, Blood 86: 3745, 1995;
Laver JH, Exp Hematol 23: 1515, 1995;
Berardi AC, Science 267: 104, 1995;
Kawashima I, Blood 87: 4136, 1996;
Leemhuis T, Exp Hematol 24: 1215, 1996;
Civin CI, Blood 88: 4102, 1996;
Larochelle A, Nature Med 2: 1329, 1996;
Tajima S, J Exp Med 184: 1357,1996;
Sakabe H, Stem Cells 15: 73, 1997;
Sakabe H, Leukemia 12: 728, 1998; and
Harada M, et al., Atarashii Zouketukansaibou Isyoku (New approach for hematopoietic stem cell transplantation), Nankodo, 1998, p. 9-23.

For example, mouse bone marrow cells are obtained by collecting bone marrow cells from femur and tibia by flushing, removing erythrocytes with Lysing buffer (0.38% NH₄Cl in Tris-HCl (pH 7.65)), and then removing mature T cells by antibody-complement reaction using anti-Thy1.2 antibody and rabbit complements (Tomita Y, Mayumi H, Eto M, Nomoto K. J Immunol. 1990; 144: 463-473). Alternatively, they are obtained by removing lineages from collected bone marrow cells by beads or centrifugation using antibodies such as anti-B220 (B cell) antibody, anti-CD3 (T cell) antibody, anti-Gr-1 (granulocyte) antibody, anti-Mac-1 (macrophage) antibody, and anti-DX-5 (NK cell) antibody (Sato T, Laver JH, Ogawa M. Blood. 1999; 94: 2548-2554).

When human hematopoietic cells are collected from the bone marrow, a few milliliters of bone marrow fluid is collected per collection from three to five sites on both of the right and left iliac bones (to avoid peripheral blood contamination) in a prone position under general anesthesia. The target amount of bone marrow collected is 3 (2 to 5) x 10⁸ cells/kg (body weight of a patient) for allogeneic transplantation, and 1 x 10⁸ to 3 x 10⁸ cells/kg for autologous transplantation. Due to contamination with bone tissues or the like, the cells are passed through a mesh and stored in a bag. At the time of bone marrow transplantation, the cells are administered after passing through a coarse filter. In the present invention, the lentiviral vectors can be introduced into bone marrow fluid collected as described above, groups of cells comprising the hematopoietic stem cells isolated from bone marrow fluid, or isolated hematopoietic stem cells.

It is preferable to select human lymphocyte antigen (HLA)-compatible donors for allogeneic transplantation. Transplantation using HLA-incompatible donors has become possible due to establishment of GVHD prevention methods, development of novel immunosuppressants, and techniques for purifying CD34-positive cells, and so on. Of the three pairs (six) antigens for HLA-A, -B, and -DR, preferably four or more, more preferably five or more, even more preferably all match with the recipient's. In the case of ABO major-incompatible transplantation, the cells are used after removing erythrocytes by centrifugation. In the case of ABO minor incompatibility, the cells are used after plasma is removed by centrifugation. (See Morishita et al, "Zouketsu Kansaibou Isyoku Manual (Manual for Hematopoietic Stem Cell Transplantation)" 2nd Ed., Kodera and Saito editorial supervisors, Nihon-Igakukan, Tokyo, (1999) p. 260-277).

When peripheral blood stem cells are collected from the peripheral blood, hematopoietic stem cells can be collected by subcutaneously injecting G-CSF once or twice a day at a daily dose of 400 µg/m² (or 10 µg/kg) for five days or every day until the collection is completed, and separating hematopoietic stem cells from peripheral blood (vein) using a blood cell separator once to three times from day 4 to day 6 after the start of administration (see documents describing guidelines for hematopoietic stem cell transplantation on the webpage of The Japan Society for Hematopoietic Cell Transplantation (Revised 3rd edition, April 21, 2003)). For recruitment of peripheral blood stem cells (PBSC), see the following references: Harada M et al., J. Hematother 5: 63-71, 1996; Waller CF et al., Bone Marrow Transplant 18: 279-283, 1996; Anderlini P et al., Blood 90: 903-908, 1997; "Atarashi Zouketsu Kansaibou Isyoku (New Approach for hematopoietic stem cell transplantation)", Harada M et al., eds., Nankodo, 1998, p. 67-72; and "Zouketsu Kansaibou Isyoku Manual (Manual for Hematopoietic Stem Cell Transplantation)", Revised 3rd Ed., Nagoya BMT group, Eds., 2004, p. 237-240.

G-CSF used for the recruitment includes not only the wild-type proteins, but also derivatives in which the N terminus has been modified (nartograstim and such) and modified proteins in which sugar chains have been added (lenograstin and such). Furthermore, G-CSF may be used in combination with other hematopoietic factors. For example, GM-CSF, IL-3, or SCF can be used in combination with G-CSF (Huhn RD et al., Exp Hematol 24: 839-847, 1996; Begley CG et al., Blood 90: 3378-3389, 1997; Lane TA et al., Blood 85: 275-282, 1995). Aspirin may be administered to alleviate systemic symptoms such as back pain, bone pain, and fever, during the period of G-CSF administration.

From the peripheral blood stem cells obtained above, human peripheral blood hematopoietic stem cells (CD34⁺ cells) can be obtained by isolating and collecting CD34-positive cells by the Isolex system (Baxter and others) and CliniMACS (AmCell) for concentrating CD34-positive cells using CD34 antibody magnetic beads; methods for concentrating through avidin columns (CEPRATE; Cellpro); methods comprising immobilizing CD34 antibodies in a flask, allowing reactive cells to adhere to the flask, and washing off the remaining cells (CELLECTOR; AIS); or such (see Morishita et al., "Zouketsu Kansaibou Isyoku Manual (Manual for Hematopoietic Stem Cell Transplantation)" 2nd Ed., Kodera and Saito, editorial supervisors, Nihon-Igakukan, Tokyo, (1999) p. 260-277).

In human cord blood cell transplantation, necessary hematopoietic stem cells are obtained by adding an erythrocyte sedimentation reagent blood cells to collected cord blood, separating each blood fraction according to weight, and removing plasma and erythrocyte fractions which are unnecessary at the time of transplantation. Then, CD34-positive cord blood hematopoietic stem cells can be obtained by purifying CD34-positive cells by the Isolex system (Baxter), CliniMACS (AmCell), concentration methods using avidin columns (CEPRATE; Cellpro), methods comprising immobilizing an CD34 antibody in a flask, allowing reactive cells to adhere to the flask, and washing off the remaining cells (CELLECTOR; AIS), or such a method (see Morishita et al., "Zouketsu Kansaibou Isyoku Manual (Manual for Hematopoietic Stem Cell Transplantation)" 2nd Ed., Kodera and Saito, editorial supervisors, Nihon-Igakukan, Tokyo, (1999) p. 661-680).

Isolated hematopoietic stem cells can be cultured, for example, in a medium supplemented with SCF, IL-3, GM-CSF, G-CSF, and Epo by the methylcellulose method (Sonoda Y. et al., Blood 84: 4099-4106, 1994; Kimura T. et al., Blood 90: 4767-4778, 1997). Hematopoietic cells are added at about 1 x 10² to 1 x 10⁴ cells/ml. The cells are cultured, for example, at 37°C under 5% CO₂ and 5% O₂. However, the culture condition may be appropriately adjusted.

In autologous transplantation, peripheral blood stem cell transplantation is commonly selected for convenience; however, autologous bone marrow transplantation can also be selected. Typically in this case, after confirming normal hematopoiesis in bone marrow, bone marrow is collected from the iliac bone and sternum under general anesthesia during the period of hematopoietic recovery in which the influence of chemotherapy is low. The target cell number is about 3 x 10⁸ to 5 x 10⁸ nucleated bone marrow cells/kg. Since cell suspensions obtained by a continuous blood component separator contain very few granulocytes and erythrocytes, the process of mononuclear cell separation can be omitted. When the sample contains many granulocytes and erythrocytes, mononuclear cells can be separated by Ficoll density centrifugation. In the case of bone marrow fluid, for removal of granulocytes and erythrocytes and concentration, mononuclear cells can be separated by Ficoll treatment or using a blood separator.

When collected cells are stored frozen, cells are suspended in RPMI1640 medium containing 10% autoserum and 10% DMSO, frozen in a programmed freezer, and stored in liquid nitrogen. The cell concentration can be adjusted to 2 x 10⁷ to 6 x 10⁷ cells/ml. When cells are stored for a relatively short period, the cell suspension (at a concentration of 1 x 10⁸ cells/ml or less) can be mixed with an equal volume of ice-cold storage liquid to obtain a final concentration of 6% Hydroxyethyl Starch (HES), 5% DMSO, and 4% albumin, and stored frozen in a deep freezer at -80°C (Knudsen LM et al., J. Hematother. 5: 399-406, 1996).

The number of CD34-positive cells required for safe autologous transplantation is about 2 x 10⁶ cells/kg (Schots R et al., Bone Marrow Transplant. 17: 509-515, 1996; Zimmerman TM et al., Bone Marrow Transplant. 15: 439-444, 1995). The number of CD34-positive cells can be measured by standard two-color flow cytometry. Specifically, erythrocytes are removed from bone marrow cells or peripheral apheresis cells by hemolytic treatment, and cytometry is developed using forward scatter and an anti-CD45 antibody, by setting the gate to exclude erythrocytes and platelets. Next, cytometry is developed with this gate using side scatter and an anti-CD34 antibody, and the number of cells in the fraction free of nonspecifically-reacting portion such as neutrophils is calculated as a percentage of the total number of cells. The total number of CD34-positive cells can be calculated from this value and the number of blood cells previously measured with a blood cell counter.

The number of viable stem cells contained in the frozen and stored cells can be determined by counting CFU-GM using colony formation assay. The standard CFU-GM required for transplantation is typically 1 x 10⁵ to 2 x 10⁵ cells/kg.

Lentiviral vectors carrying a blood coagulation factor gene can be introduced into hematopoietic stem cells by contacting an agent of the present invention for treating blood coagulation abnormalities with hematopoietic stem cells prepared as described above or with a group of cells comprising hematopoietic stem cells. The lentiviral vectors be contacted with hematopoietic stem cells within the body (*in vivo*) or outside the body (*in vitro* or *ex vivo*). The lentiviral vectors can be introduced into cells, for example, by contacting the two in a desired physiological aqueous solution such as culture medium, physiological saline, blood, plasma, serum, and body fluid. The efficiency of lentiviral vector infection can be increased by contacting the two in the presence of polybrene or Retronectin.

When the vector is contacted with hematopoietic stem cells, the multiplicity of infection (MOI; the number of infecting viruses per cell) can be adjusted to 1 to 500. MOI is typically 2 to 300, for example, 3 to 200, preferably 5 to 100, more preferably 7 to 70. Under such a condition, the lentiviral vectors are introduced into hematopoietic stem cells in a very short time. Specifically, the contact time of the two can be one minute or more, typically three minutes or more, for example, five minutes or more, preferably ten minutes or more, or 20 minutes or more. The two can be contacted, for example, for about 1 to 60 minutes, more specifically, for about five to 30 minutes. Of course, the two may be contacted for a longer period, for example, for several days or longer.

Hematopoietic stem cells into which the vector has been introduced can be combined with a desired pharmaceutically acceptable carrier or medium and used as a composition for hematopoietic stem cell transplantation. The "pharmaceutically acceptable carrier or medium" is not particularly limited, as long as it is a solution that can be used to suspend viable cells. It includes, for example, phosphate buffered saline (PBS), sodium chloride solution, Ringer's solution, and culture media.

Furthermore, the compositions for hematopoietic stem cell transplantation may contain hematopoietic cells that have not been introduced with the vector. The efficiency of grafting hematopoietic cells may be increased by combining hematopoietic cells into which the vector has been introduced with those that have not been introduced with the vector. For example, the mixing ratio of hematopoietic cells introduced with the vector to hematopoietic cells not introduced with the vector may be appropriately adjusted to between 1:10 and 10:1, without being limited thereto.

The present invention relates to methods for inducing the differentiation of hematopoietic cells introduced with the above-described lentiviral vector into megakaryocytes or platelets. These methods comprise the steps of: (a) contacting hematopoietic stem cells with a lentiviral vector carrying a polynucleotide encoding a blood coagulation factor operably linked to a platelet-specific promoter; and (b) injecting the hematopoietic stem cells of step (a) into an animal.

Alternatively, the present invention relates to methods for treating blood coagulation abnormalities, which comprise the steps of:
(1) infecting hematopoietic stem cells with a lentiviral vector comprising a platelet-specific promoter and a polynucleotide encoding a blood coagulation factor operably linked to the promoter; and
(2) administering the hematopoietic stem cells of step (1) into a patient with blood coagulation abnormality.

In the present invention, there is no limitation on the origin of hematopoietic stem cells used for treating blood coagulation abnormalities. When hematopoietic stem cells are collected from patients who are subject of the treatment, engraftment is easy and there is no risk of GVHD or such. However, when immunosuppressants or the like are used, hematopoietic stem cells of different individuals can also be applied in the present invention. In the therapeutic methods of the present invention, the origin of hematopoietic stem cells is not limited as long as the cells can differentiate into platelets. Specifically, cells containing either or both of bone marrow stem cells and peripheral blood hematopoietic stem cells, and the like can be used. For example, bone marrow cells are preferred hematopoietic stem cells in the therapeutic methods of the present invention.

In the present invention, hematopoietic stem cells that are suitably cultured after infection with the lentiviral vectors can also be administered to patients. The therapeutic efficiency for blood coagulation abnormalities by the present invention can be improved, for example, by culturing the above-mentioned lentiviral vector-infected hematopoietic stem cells in a medium for inducing differentiation and inducing the differentiation into megakaryocytes. In general, hematopoietic stem cells have the ability to differentiate into not only megakaryocytes (platelet precursor cells), but also erythrocytes and leukocytes. Accordingly, the lentiviral vector-infected cells may differentiate into not only megakaryocytes, but also erythrocytes or leukocytes by simply returning the hematopoietic stem cells to the body.

In the present invention, however, a blood coagulation factor is expressed under the control of a platelet-specific promoter, and thus the blood coagulation factor may not be expressed in blood cells other than platelets. Thus, the therapeutic effect of the present invention is improved by increasing the proportion of lentiviral vector-infected hematopoietic stem cells differentiating into megakaryocytes. Culture conditions that induce differentiation into megakaryocytes are known.

For example, bone marrow collected from patients is cultured for about 24 hours in IMDM with the composition shown below. After culture, it is contacted with the lentiviral vector. After 10 to 24 hours, the cells are washed and administered to patients. Retronectin is not essential for the culture.
IMDM 1% serum albumin
200 µg/ml of transferrin
10 µg/ml of insulin
100 ng/ml of stem cell factor (SCF)
10 ng/ml to 100 ng/ml of thrombopoietin (TPO)
100 ng/ml of interleukin-6 (IL-6)
100 ng/ml of Flt-3 ligand (Flt-3L)
400 ng/ml of soluble IL-6 receptor (sIL-6R)

The serum albumin in the above composition may be, for example, fatty acid-free bovine serum albumin (fatty acid-free BSA) or virus-free human serum albumin preparation. Moreover, the amount of TPO added can be, specifically, for example, 100 ng/ml (Mostoslavsky et al. Mol. Ther. 11, 932-940, 2005).

The elements used in the methods of present invention for treating blood coagulation abnormalities can be combined in advance and provided as a kit for treatment. Thus, the present invention relates to kits for treating blood coagulation abnormalities, which comprise the elements described below.
a: a lentiviral vector comprising a platelet-specific promoter and a polynucleotide encoding a blood coagulation factor operably linked to the promoter; and
b: a reagent for collecting hematopoietic stem cells.

In the therapeutic kits of the present invention, the above lentiviral vectors can be separately packaged in amounts required for the treatment. For example, in the case of cord blood transplantation, hematopoietic stem cells required for engraftment is generally believed to be 2 x 10⁷ to 3 x 10⁷ cells/kg. Thus, when a person weighs 50 kg, it is estimated in calculation that 1 x 10⁹ to 1.5 x 10⁹ cells are needed. When the MOI and functional titer are 10 and 1 x 10¹⁰ /ml, respectively, the volume of viral solution is 1 to 1.5 ml. If the MOI is 30, the volume of the solution is simply tripled.

Alternatively, for patients with known severe combined immunodeficiency, CD34-positive cells are separated from bone marrow cells and 14 x 10⁶ to 38 x 10⁶ cells/kg are administered (N Engl J Med. 2002 Apr 18; 346(16):1185-93). Under the condition indicated in this report, among the administered cells, the number of cells introduced with a gene was 5 x 10⁶ to 20 x 10⁶ cells/kg. If the present invention is carried out under the same condition, 1 x 10⁹ cells will be required, assuming that about 2 x 10⁷ cells/kg, all of which infected with the lentiviral vector, are administered to a 50 kg human. The volume of viral solution required for this treatment is 1 ml when the MOI is 10. Thus, at an MOI of 10, the fluid volume for the lentiviral vector in the therapeutic kits of the present invention can be, for example, 0.05 to 50 ml, typically 0.5 to 20 ml, specifically 0.8 to 10 ml, or 0.8 to 2 ml.

Furthermore, the present inventors' finding suggests that a sufficient therapeutic effect can be expected by allowing the lentiviral vector to infect not all but about half of the cells administered in the present invention. Thus, the amount of lentiviral vector used can be further reduced. Specifically, the kits can be constituted using the same volume of viral solution with an MOI of about 15. Alternatively, a therapeutic effect can be expected even when the number of cells to be infected is reduced, for example, to about half of the number under the above condition.

Meanwhile, the reagents for collecting hematopoietic stem cells, which also constitute the therapeutic kits of the present invention, are reagents used to obtain hematopoietic stem cells to be infected with the above-described lentiviral vector. Methods for isolating human hematopoietic stem cells are known. Specifically, for example, antibodies that bind to cellular markers for isolating hematopoietic stem cells are included in the reagents for obtaining hematopoietic stem cells.

The therapeutic kits of the present invention can additionally include a culture medium for inducing the differentiation of hematopoietic stem cells into megakaryocytes. As described above, the therapeutic efficiency of the present invention can be improved by increasing the proportion of lentiviral vector-infected hematopoietic stem cells differentiating into megakaryocytes. Culture media that can induce the differentiation of hematopoietic stem cells into megakaryocytes are known. For example, culture media containing the cytokines listed below are useful in cultures for inducing the differentiation of bone marrow cells.
transferrin;
insulin;
stem cell factor;
thrombopoietin;
interleukin-6;
Flt-3 ligand; and
soluble interleukin-6 receptor.

Alternatively, the therapeutic methods of the present invention can also be conducted by transplanting hematopoietic stem cells (donor) of different origins into a patient (recipient). Specifically, hematopoietic stem cells collected from a donor are infected with the lentiviral vector. The hematopoietic stem cells obtained are cultured if necessary, and then administered to patients with blood coagulation abnormality. Under normal conditions, the hematopoietic stem cells have a high risk to be eliminated by the patient's immune response. However, it has been demonstrated that a certain number of hematopoietic stem cells engraft by preliminary immunosuppressive treatment of the recipient. Such transplantation methods which do not involve a total destruction of bone marrow tissues are called mini-transplantation or the like. Thus, the present invention's methods for treating blood coagulation abnormalities can also be performed using hematopoietic stem cells from donors.

When the therapeutic methods of the present invention are conducted using hematopoietic stem cells from donors, hematopoietic stem cells infected with the lentiviral vector can be provided as agents for treating blood coagulation abnormalities. Thus, the present invention relates to hematopoietic stem cells infected with lentiviral vectors comprising a platelet-specific promoter and a polynucleotide encoding a blood coagulation factor operably linked to the promoter.

Alternatively, the present invention provides agents for treating blood coagulation abnormalities, which comprise as an active ingredient hematopoietic stem cells infected with lentiviral vectors comprising a platelet-specific promoter and a polynucleotide encoding a blood coagulation factor operably linked to the promoter. Furthermore, the present invention relates to the use of hematopoietic stem cells infected with lentiviral vectors comprising a platelet-specific promoter and a polynucleotide encoding a blood coagulation factor operably linked to the promoter in the production of agents for treating blood coagulation abnormalities.

Furthermore, in the present invention, megakaryocytes or platelets that have differentiated from lentiviral vector-infected hematopoietic stem cells can also be provided as agents for treating blood coagulation abnormalities. Thus, the present invention provides either or both of megakaryocytes and platelets that have differentiated from hematopoietic stem cells infected with lentiviral vectors comprising a platelet-specific promoter and a polynucleotide encoding a blood coagulation factor operably linked to the promoter. Herein, these megakaryocytes and platelets are referred to as cells introduced with a blood coagulation factor.

Alternatively, the present invention provides agents for treating blood coagulation abnormalities, which comprise as an active ingredient the above-mentioned cells introduced with a blood coagulation factor. Furthermore, the present invention relates to the use of the above-mentioned cells introduced with a blood coagulation factor in the production of agents for treating blood coagulation abnormalities.

In addition, the present invention relates to pharmaceutical packages for treating blood coagulation abnormalities, which comprise hematopoietic stem cells infected with lentiviral vectors comprising a platelet-specific promoter and a polynucleotide encoding a blood coagulation factor operably linked to the promoter, and instructions stating that the hematopoietic stem cells are used to treat blood coagulation abnormalities. Likewise, the present invention relates to pharmaceutical packages for treating blood coagulation abnormalities, which comprise the above-mentioned cells introduced with a blood coagulation factor and instructions stating that the above-mentioned cells introduced with a blood coagulation factor are used to treat blood coagulation abnormalities.

All prior art references cited herein are incorporated by reference into this description.

### Examples

### <Materials and Methods>

### (1) Mice

Hemophilia A mice with targeted disruption of exon 16 of the FVIII gene were provided by H. H. Kazazian Jr. (the University of Pennsylvania, Philadelphia, PA, U.S.A.) (Bi, L., Lawler, A.M., Antonarakis, S.E., High, K.A., Gearhart, J.D., and Kazazian, H. H. Jr. (1995) Targeted disruption of the mouse Factor VIII gene produces a model of haemophilia A. Nat. Genet. 10, 119-121). C57BL/6 (B6-Ly5.2) mice were purchased from Japan SLC Inc. (Shizuoka, Japan). C57BL/6 mice in which a gene has been introduced into the Ly5 locus (B6-Ly5.1) were purchased from Sankyo Labo Service Co. (Tsukuba, Japan). Animal care was performed according to the standard guidelines of the Jichi Medical University Animal Care Committee and related Committees (Madoiwa, S., Yamauchi, T., Hakamata, Y., Kobayashi, E., Arai, M., Sugo, T., Mimuro, J., and Sakata, Y (2004) Induction of immune tolerance by neonatal intravenous injection of human factor VIII in murine hemophilia A. J. Thromb. Haemost. 2, 754-762).

### (2) Cytokines and antibodies

Recombinant human thrombopoietin (TPO) and recombinant human stem cell factor (SCF) were provided by Kirin Brewery Co. (Gunma, Japan). The materials listed below were obtained from the indicated suppliers.
Recombinant human IL-6 (IL-6), recombinant human soluble IL-6 receptor (sIL-6R), and recombinant human Flt-3 ligand (Flt3-L) (PeproTech EC, London, England);
Recombinant human IL-3 (IL-3) (TECHNE, Minneapolis, MN);
Anti-mouse c-kit monoclonal antibody (MoAb) (Clone 2B8), anti-mouse Sca-1 MoAb (Clone D7), anti-mouse Ly5 (CD45) MoAb (Clone 30-F11), and anti-mouse Ly5.1 (CD45.1) MoAb (Clone A20) (BD Pharmingen, CA);
Anti-mouse GPIbα MoAb (Clone Xia.G5) (Emfret Analytics GmbH, Wurzberg, Germany), anti-human GPIIb/IIIa MoAb (Clone 5B12), anti-human GPIbα MoAb (Clone AN51), and anti-human CD34 MoAb (Clone BIRMA-K3) (DakoCytomation, Glostrup, Denmark).

### (3) Cell culture

The human megakaryoblastic cell line UT-7/TPO was provided by Dr. Norio Komatsu (the University of Yamanashi, Yamanashi, Japan) (Komatsu, N., Kunitama, M., Yamada, M., Hagiwara, T., Kato, T., Miyazaki, H., Eguchi, M., Yamamoto, M., and Miura, Y. (1996) Establishment and characterization of the thrombopoietin-dependent megakaryocytic cell line, UT-7/TPO. Blood 87, 4552-4560). The cells were cultured in Iscove's modified Dulbecco medium (IMDM) supplemented with 10% fetal bovine serum (FBS) and 10 ng/ml TPO.

Human umbilical vein endothelial cells (HUVECs) were obtained and maintained as described previously (Hisano, N., Yatomi, Y, Satoh, K., Akimoto, S., Mitsumata, M., Fujino, M.A., and Ozaki, Y (1999) Induction and suppression of endothelial cell apoptosis by sphingolipids: a possible in vitro model for cell-cell interactions between platelets and endothelial cells. Blood 93, 4293-4299).

### (4) Megakaryocyte differentiation

Human CD34⁺ cells derived from cord blood were isolated using the AutoMACS magnetic cell separation system (Miltenyi Biotec., Auburn, CA) according to the manufacturer's instructions. The purity of the isolated CD34⁺ cells was higher than 90% (data not shown). The CD34⁺ cells were grown in IMDM containing 1% fatty acid-free BSA, 200 µg/ml iron-saturated human transferrin, and 10 µg/ml human recombinant insulin, and supplemented with 50 ng/ml TPO and 10 ng/ml IL-3; and cells differentiated into megakaryocytes (Majka, M., Rozmyslowicz, T., Lee, B., Murphy, S.L., Pietrzkowski, Z., Gaulton, G.N., Silberstein, L., and Ratajczak, M.Z. (1999) Bone marrow CD34+ cells and megakaryoblasts secrete -chemokines that block infection of hematopoietic cells by M-tropic R5 HIV. J. Clin. Invest. 104, 1739-1749). 75 to 90% of the cells were positive for GPIIb/III after 14 days of culture (Fig. 2C).

### (5) Construction of luciferase reporter plasmid, transient transfection, and luciferase assay

The DNA fragments listed below, which were assumed to have the maximum promoter activity, were amplified by PCR using human genomic DNA as a template.
GPIIb promoter: -554 to +28 (Prandini, M.H., Uzan, G., Martin, F., Thevenon, D., and Marguerie, G. (1992) Characterization of a specific erythromegakaryocytic enhancer within the glycoprotein IIb promoter. J. Biol. Chem. 267, 10370-10374);
GPIbα promoter: -254 to +330 (Hashimoto, Y, and Ware, J. (1995) Identification of essential GATA and Ets binding motifs within the promoter of the platelet glycoprotein Ib gene. J. Biol. Chem. 270, 24532-24539); and
GPVI promoter: -320 to +28 (Holmes, M.L., Bartle, N., Eisbacher, M., and Chong, B.H. (2002) Cloning and analysis of the thrombopoietin-induced megakaryocyte-specific glycoprotein VI promoter and its regulation by GATA-1, Fly-1, and Sp1. J. Biol. Chem. 277, 48333-48341).

After subcloning into PCR-Blunt-TOPO (Invitrogen, Carlsbad, CA), fragments were cloned into pGL3-basic, a promoterless luciferase plasmid (Promega, Madison, CA). 500,000 cells (UT-7/TPO and CD34⁺-derived megakaryocytes) were placed in IMDM (without FBS or growth factors) in each well of 6-well plates and transfected with 4 µg of plasmid DNA using DMRIE-C reagent (Invitrogen). After four hours, an equal volume of IMDM supplemented with 2x growth factors was added to each well. The cells were incubated at 37°C for 48 hours, and then luciferase activity was assayed according to the manufacturer's instructions (Luciferase Assay System; Promega).

### (6) Construction and production of SIV vector

A replication-incompetent self-inactivating SIV vector was constructed as described previously (Nakaj ima, T., Nakamaru, K., Ido, E., Terao, K., Hayami, M., and Hasegawa, M. (2000) Development of novel simian immunodeficiency virus vectors carrying a dual gene expression system. Hum. Gene. Ther. 11, 1863-1874). The full-length human FVIII (hFVIII) cDNA was provided by Dr. J. A. van Mourik (blood coagulating effect, Sanquin, Amsterdam, the Netherlands). Then, a human B domain-deleted (BDD) FVIII (hBDD-FVIII) cDNA was prepared by PCR-based mutagenesis as described previously (Lind, P., Larsson, K., Spira, J., Sydow-Backman, M., Almstedt, A., Gray, E., and Sandberg, H. (1995) Novel forms of B-domain-deleted recombinant factor VIII molecules. Construction and biochemical characterization. Eur. J. Biochem. 232, 19-27).

The eGFP gene (or the hFVIII gene) driven by a cytomegalovirus (CMV) promoter was inserted between the LTR-containing elements (U3, R, and U5) of the SIV-derived vector to obtain SIV-pCMV-eGFP/hFVIII. Likewise, the eGFP gene (or the hFVIII gene) driven by a GPIbα promoter was inserted between the LTR-containing elements (U3, R, and U5) of the SIV-derived vector to obtain SIV-pGPIbα-eGFP/hFVIII (Fig. 2A).

293 T cells were transfected with the gene transfer plasmids, together with three packaging plasmids (encoding gag-pol, rev, and VSV-G env) using lipofectamine Plus Reagent (Invitrogen). After 12 hours, the culture medium was changed and viral particles were collected. The culture medium was collected after 48 hours, and the viral particles were concentrated by centrifugation. The transduction unit of the eGFP-containing SIV vector was measured by infecting 293 cells, and then eGFP expression was measured by FACS analysis. The averaged infectivity of the SIV-CMV eGFP vector was in a range of 2 x 10⁸ to 5 x 10⁸ TU/ml. To compare viral infectivity between various promoters or target genes, viral particle titers were simultaneously measured by real-time quantitative RT-PCR. Viral RNAs were isolated using the QIAamp Viral RNA Mini kit (QIAGEN, Valencia, CA). The isolated RNAs were reverse-transcribed using SuperScript II (Invitrogen).

Replication products of the vector-specific posttranscriptional regulatory element derived from the sequence of woodchuck hepatitis virus (WPRE) was measured by real-time quantitative PCR using the QuantiTectProbe PCR system (Qiagen) to quantify the vector particles. The WPRE sequence was amplified using WPRE forward primer:
5'-GCTTTCATTTTCTCCTCCTT-3' (SEQ ID NO: 11) and WPRE reverse primer:
5'-GGCCACAACTCCTCATAA-3' (SEQ ID NO: 12). The sequence of FAM-labeled probe was 5'-ATCCTGGTTGCTGTCTC-3'(SEQ ID NO: 13).

PCR was begun with an initial incubation step of 15 minutes at 95°C. The thermal cycle was composed of 45 cycles of: 94°C for 15 seconds, 56°C for 30 seconds, and 76°C for 30 seconds. The reporter fluorescent signal of the specific probe was detected during the PCR annealing steps by the ABI PRISM 7700 Sequence Detector system (PE Applied Biosystems, Foster City, CA). The averaged viral particle titer of the SIV-based vector was in a range of 1 x 10¹⁰ to 2 x 10¹⁰ TU/ml. The infectivity (multiplicity of infection (MOI)) of the SIV vector was calculated from the ratio of the particle titer to SIV-CMV-eGFP.

To transduce UT-7/TPO and CD34⁺-derived megakaryocytes with the SIV vectors, 1 x 10⁵ cells were resuspended in 100 µl of culture medium containing 8 µg/ml polybrene. The cells were transduced with the SIV vectors at various MOIs indicated by the 24-hour test. Then, the cells were resuspended in 300 µl of PBS containing 0.5% BSA and 2 mM EDTA, and eGFP-positive cells were analyzed by FACS analysis.

### (7) Isolation of hematopoietic stem cells and viral transduction

Cells expressing cell lineage markers (B220, CD5, CD11b, Gr-1, and Ter-119) were removed from bone marrow cells obtained from mouse femur and tibia by magnetic separation using the Lineage Cell Depletion kit (Miltenyi Biotec). Then, Sca-1⁺ and c-kit⁺ cells (KSL) were isolated by FACS (FACSAria cell sorter, Becton Dickinson).

When newly isolated KSL cells were directly infected with the SIV vectors, PI-positive cells (dead cells) increased after transduction and the transduction efficiency was decreased (data not shown). Thus, culture conditions of KSL cells were assessed to improve SIV transduction and cell viability. When isolated KSL cells were co-cultured with IL-3, IL-6, and SCF, the transduction efficiency was lower (25% to 35% at an MOI of 30) and PI-positive cells were significantly increased (20% to 30%) for SIV transduction of KSL cells with the eGFP gene (data not shown).

Meanwhile, when KSL cells were co-cultured with SCF, IL-6, sIL-6R, Flt-3L, and TPO for 3 to 7 days, a high level of eGFP expression was obtained (see Fig. 3). PI-positive cells after eGFP transduction were significantly decreased (8 to 12%). Thus, before viral transduction, KSL cells were pre-cultured in IMDM containing 1% fatty acid-free BSA, 200 µg/ml transferrin, and 10 µg/ml insulin, and supplemented with the following substances:
100 ng/ml SCF;
10 ng/ml TPO;
100 ng/ml IL-6;
100 ng/ml Flt-3L; and
400 ng/ml sIL-6R.

The cells were infected with SIV in plates coated with 50 µg/ml RetroNectin (TakaraBio, Tokyo, Japan). The cultured KSL cells (1 x 10⁵ or 1 x 10⁶ cells) were resuspended in 100 µl or 1 ml of 10% FBS-containing IMDM. The cells were introduced with the SIV vectors at the various MOIs determined at the 12^{th} hour of incubation with the same combination of cytokines as described above, and then incubated at 37°C. The use of polybrene (up to 4 µg/ml) did not bring significant improvement of transduction efficiency for mouse KSL and human CD34⁺ cells (data not shown). Therefore, transduction of stem cells was carried out without polybrene. Then, cells were resuspended in 100 µl of PBS containing 1% bovine serum albumin (BSA) for transplantation, or incubated for the period (days) shown in the FACS analysis result.

### (8) Stem cell transplantation

Bone marrow cells were obtained from B6-Ly5.1 so that donor cells could be distinguished from recipient cells. Recipient mice (B6-Ly5.2; 8- to 12-week old) were irradiated at a single lethal dose of 9.5 Gy (⁶⁰Co, Gamma Cell; Nortion International, ON, Canada). 100,000 cultured KSL cells derived from SIV-transduced or non-transduced B6-Ly5.1 were injected, along with 5 x 10⁵ newly isolated unfractionated Ly5.2 bone marrow cells as competitor cells. To assess the bone marrow reconstitution, peripheral blood was collected from the retro-orbital sinus using heparin-coated micropipette and analyzed for the percentages of Ly5.1 (donor-derived) lymphocytes and bone marrow cells by flow cytometry. According to the transplantation procedure of the present inventors, transplantation of Ly5.1 cells accounted for 40% to 55% of lymphocytes and bone marrow cells four months after transplantation.

### (9) Detection of hFVIII transgene transcription

The transcription of the hFVIII transgene was detected by RT-PCR (Kikuchi, J., Mimuro, J., Ogata, K., Tabata, T., Ueda, Y., Ishiwata, A., Kimura, K., Takano, K., Madoiwa, S., Mizukami, H., Hanazono, Y, Kume, A., Hasegawa, M., Ozawa, K., and Sakata, Y (2004) Sustained transgene expression by human cord blood derived CD34+ cells transduced with simian immunodeficiency virus agmTYO1-based vectors carrying the human coagulation factor VIII gene in NOD/SCID mice. J. Gene. Med. 6, 1049-1060). RNAs were isolated from mouse organs using RNA isolation kit (RNeasy Protect kit; QIAGEN). The RNA samples were analyzed by RT-PCR using a pair of primers for hFVIII (17) and the RT-PCR kit (SuperScript One-step RT-PCR System, Invitrogen). A pair of primers for mouse GAPDH mRNA (R&D Systems, Minneapolis, MN) was used in the control RT-PCR experiments.

The expression of SIV vector-derived mRNAs was quantified by real-time quantitative RT-PCR using the QuantiTect Probe RT-PCR system (Qiagen). The amount of analyzed RNA was estimated using Mouse GAPDH (mGAPDH) Control reagent (Qiagen). A standard curve was established by analyzing a series of serial dilutions of the SIV gene transfer vector. 50 ng of purified RNA extracted from tissues was used as template. The amount of WPRE sequence was determined for each sample by estimating the amount of WPRE sequence as compared with the standard control curve and dividing the WPRE sequence copy number by the mGAPDH sequence copy number.

### (10) Immunohistochemistry

Bone marrow cells attached to glass slides were fixed in PBS containing 3% paraformaldehyde using Cytospin3 (Shandon, ThermoShandon, PA). Then, the cells were permeabilized with 0.2% Triton X-100. After blocking with 1% BSA, the samples were incubated with a biotin-conjugated anti-FVIII polyclonal antibody at 4°C for two hours. After incubation, the samples were washed with PBS. Then, the samples were incubated with streptavidin-conjugated Alexa 594 (Molecular Probes, Eugene, OR) and a FITC-labeled anti-mouse GPIbα monoclonal antibody. The resulting immunofluorescent stain was observed and photographed using a fluorescent microscope with attached camera.

For immunohistochemical detection of hFVIII molecules in mouse tissues, spleens were fixed in phosphate buffered saline (PBS) containing 4% paraformaldehyde at 4°C for two hours. The spleens were incubated in PBS containing sucrose (10% to 30%), and then frozen in the presence of OCT compound in dry ice/ethanol. Sections were prepared from tissues frozen at -25°C and adhered onto polylysine-coated glass slides. For detection of hFVIII, the tissue sections were blocked in PBS containing 1% rabbit serum and Triton-X100 (0.1%), and incubated with sheep anti-human FVIII polyclonal antibody (Cedarlane Labs, Homby, Ontario, Canada) at 4°C for 16 hours. After washing with PBS, the sections were incubated with biotin-conjugated rabbit anti-sheep IgG antibody, followed by ABC reagent (Vectastain ABC Elite kit; Vector, Burlingame, CA) and DAB kit (Vector).

### (11) Measurement of hFVIII antigen, neutralizing antibodies, and phenotypic alteration

hFVIII antigen was measured using an anti-hFVIII-specific ELISA kit (Affinity Biological, Hamilton, ON, Canada) and compared with the measured value for human standard plasma pool. To inhibit platelet activity, whole blood (270 ml) was collected from the superior vena cava of anesthetized mice using a syringe containing 30 µl sodium citrate and 1 µM PGI₂. To activate platelets, 1 µM phorbol myristate acetate (PMA) and 50 µg/ml collagen were added to the blood at the time shown in the Results. After centrifugation, the platelet-depleted plasma was frozen at -80°C until analyzed for FVIII antigen. Neutralizing antibodies against hFVIII, which were induced in the mice, were analyzed by Bethesda method using the FVIII-depleted plasma and standard plasma pool (Madoiwa, S., Yamauchi, T., Hakamata, Y., Kobayashi, E., Arai, M., Sugo, T., Mimuro, J., and Sakata, Y. (2004) Induction of immune tolerance by neonatal intravenous injection of human factor VIII in murine hemophilia A. J. Thromb. Haemost. 2, 754-762). A few transplanted mice were anesthetized with diethyl ether, and the tails (1.5 cm) were cut to test the phenotypic correction. Survival of mice was observed 24 hours later.

The phenotype of hemophilia A mice used in the experiment was functional deficiency in blood coagulation. Therefore, when the phenotype is not corrected, animals whose tail has been cut will eventually die due to loss of blood. If the survival rate is improved, it can be confirmed that the Factor VIII of transduced platelets has complemented the blood coagulation function. Therefore, phenotypic correction can be confirmed by the survival of hemophilia A mice whose tail has been cut.

### <Results>

### [Example 1] Comparison of the luciferase reporter expression promoted by the platelet-specific promoter

First, the present inventors compared the activities of promoters of three platelet-specific genes, i.e. GPIIb, GPIbα, and GPVI, in megakaryocytes to achieve efficient expression of the target gene in platelets. Fig. 1A shows a schematic illustration of the platelet-specific promoters used in this study, along with regulatory factors specific to the promoters. Various promoters were compared for their promoter activities using the luciferase reporter gene. The present inventors used nucleotide regions of promoters which had the highest activity in previous studies (Prandini, M.H., Uzan, G., Martin, F., Thevenon, D., and Marguerie, G. (1992) Characterization of a specific erythromegakaryocytic enhancer within the glycoprotein IIb promoter. J. Biol. Chem. 267, 10370-10374; Hashimoto, Y, and Ware, J. (1995) Identification of essential GATA and Ets binding motifs within the promoter of the platelet glycoprotein Ib gene. J. Biol. Chem. 270, 24532-24539; Holmes, M.L., Bartle, N., Eisbacher, M., and Chong, B.H. (2002) Cloning and analysis of the thrombopoietin-induced megakaryocyte-specific glycoprotein VI promoter and its regulation by GATA-1, Fli-1, and Sp1. J. Biol. Chem. 277, 48333-48341).

The GPIbα promoter directs the strongest luciferase expression in UT-7/TPO cells, a megakaryoblastic cell line (Fig. 1B). The relative efficiency of the GPIbα promoter is even more remarkable in CD34⁺-derived megakaryocytes (Fig. 1C). According to reports, GPIb and GPVI are expressed in endothelial cells (Konkle, B.A., Shapiro, S.S., Asch, A.S., and Nachman, R.L. (1990) Cytokine-enhanced expression of glycoprotein Ib in human endothelium. J. Biol. Chem. 265, 19833-19838, Sun, B., Tao, L., Lin, S., Calingasan, N.Y, Li, J., Tandon, N.N., Yoshitake, M., and Kambayashi, J. (2003) Expression of glycoprotein VI in vascular endothelial cells. Platelets 14, 225-232). Thus, the present inventors examined whether the activities of these platelet-specific promoters were stimulated in endothelial cells.

Under conditions in which the PAI-1 promoter (Mimuro, J., Muramatsu, S., Hakamada, Y, Mori, K., Kikuchi, J., Urabe, M., Madoiwa, S., Ozawa, K., and Sakata, Y. (2001) Recombinant adeno-associated virus vector-transduced vascular endothelial cells express the thrombomodulin transgene under the regulation of enhanced plasminogen activator inhibitor-1 promoter. Gene Ther. 8, 1690-1697) efficiently directs luciferase expression, the GPIIb, GPIbα, and GPVI promoters did not direct luciferase expression in HUVECs (Fig. 1D). Thus, the present inventors decided to use GPIbα to direct platelet-specific expression of target genes in the present study.

### [Example 2] Efficient transformation of megakaryocytes by SIV-based vectors

The present inventors constructed two SIV-based lentiviral vectors carrying the eGFP gene under the control of either the CMV promoter (SIV-pCMV-eGFP) or the GPIbα promoter (SIV-pGPIbα-eGFP). The transgene located downstream of the CMV promoter (pCMV) or GPIbα promoter (pGPIbα) was inserted between the LTR-containing elements (U3, R, and U5; Fig. 2A) of a SIV-derived vector (Fig. 2A). A posttranscriptional regulatory factor derived from woodchuck hepatitis virus (WPRE) was inserted downstream of the expressed gene to increase gene expression in the transduced cells.

To investigate the transduction of eGFP gene into megakaryocytes, UT-7/TPO or CD34⁺-derived megakaryocytes were incubated for 24 hours in the presence of SIV-pCMV-eGFP or SIV-pGPIbα-eGFP at various concentrations. Both constructs efficiently transduced the eGFP gene into UT-7/TPO and CD34⁺-derived megakaryocytes (Fig. 2B). When the cells were incubated with SIV-pCMV-eGFP or SIV-pGPIb at an MOI of about 1 and 3, respectively, eGFP expression reached the half maximal level. Meanwhile, transduction using lipofection introduced the eGFP gene into only 8% to 12% of UT-7/TPO or CD34⁺-derived megakaryocytes (data not shown).

Next, the present inventors investigated whether *ex vivo* megakaryocyte differentiation influences gene expression. The expression of GPIIb and GPIb on the cell surface was investigated by flow cytometry under a condition in which cord blood-derived CD34⁺ cells are differentiated into megakaryocytes by IL-3 and TPO. While GPIb was not found on Day 0, about 18% of the cells were positive for GPIIb (Fig. 2D). Furthermore, as described previously (Lepage, A., Leboeuf, M., Cazenave, J.P., de la Salle, C., Lanza, F., and Uzan, G. (2000) The IIb 3 integrin and GPIb-V-IX complex identify distinct stages in the maturation of CD34+ cord blood cells to megakaryocytes. Blood 96, 4169-4177), GPIb was thought to be expressed at a late stage of the megakaryocyte differentiation process (Fig. 2D). The expression of eGFP promoted by the CMV promoter was not influenced by megakaryocyte differentiation (Fig, 2E). Meanwhile, under the control of the pGPIbα promoter, the expression of eGFP was promoted during differentiation of CD34⁺ cells into megakaryocytes (Fig. 2D).

### [Example 3] Establishment of efficient transduction of KSL cells carrying SIV vector

Next, the present inventors optimized the transduction protocol for KSL cells by using an SIV vector carrying the eGFP gene promoted by the CMV promoter. The efficiency of eGFP transduction in cultured KSL cells reached 60% to 80% (Fig. 3A). The plateau value for transduction was observed at an MOI of 10 to 30. One day (24 hours) after incubation with the viral vector was sufficient to achieve a high expression of the transduced gene. The expression level of eGFP then gradually decreased (Fig. 3B). The decrease of eGFP expression can be due to a reduction in cell viability. This is because PI-positive cells (dead cells) increased with time (data not shown). Thus, the present inventors cultured KSL at an MOI of 30 for 24 hours and transplanted the cells into recipient mice in the subsequent experiments.

### [Example 4] Preferential eGFP expression in platelets using SIV vectors intrinsically carrying the GPIbα promoter in vivo

To compare the strength and specificity of the CMV and GPIbα promoters and to assess SIV vector-mediated eGFP transduction *in vivo*, KSL cells transduced with SIV-pCMV-eGFP or SIV-pGPIb-eGFP were transplanted into recipient mice (Ly5.2). 1 x 10⁵ cultured KSL cells (Ly5.1) transduced with SIV-pCMV-eGFP or SIV-pGPIbα-eGFP (at an MOI of 30) were transplanted together with 5 x 10⁵ competitor cells (Ly5.2).

When KSL cells transduced with SIV-pCMV-eGFP were transplanted, eGFP expression was observed in 35% to 45% of CD45⁺ cells and 7% to 11% of platelets in the peripheral blood (Fig. 4A and B). Interestingly, the transduction by the SIV vector intrinsically carrying the GPIbα promoter resulted in efficient gene marking of platelets (16 to 27%) but not of CD45⁺ cells (Fig. 4A and B). The present inventors then analyzed bone marrow cells derived from transplanted mice using specific markers to identify macrophages, granulocytes, B lymphocytes, T lymphocytes, and erythroblasts. While eGFP was expressed in these cell lineages of mice which received KSL cells transduced with SIV-pCMV-eGFP, the GPIbα promoter did not promote eGFP expression in these cell lineages. Thus, the specificity of the activity in megakaryocytes and platelets was verified (Fig. 4C).

### [Example 5] hFVIII expression and phenotypic correction in hemophilia A mice transplanted with KSL cells transduced with SIV-pGPIbα-hFVIII

To determine whether platelet-dependent gene therapy enables sustained FVIII expression, the present inventors constructed two SIV vectors carrying an hBDD-FVIII cDNA under the control of the CMV (SIV-pCMV-hFVIII) or GPIbα promoter (SIV-pGPIbα-hFVIII). The present inventors transplanted 1 x 10⁵ transduced KSL cells into mice and then carried out a y ray irradiation. The present inventors first analyzed the levels of hFVIII gene transcripts present in organs of transplant recipients three months after transplantation. Transcript analysis of hFVIII promoted by the CMV promoter revealed that the hFVIII gene was mainly expressed in the bone marrow, and to a lesser extent in the spleen (Fig. 5A, middle panel). Interestingly, hFVIII mRNA was found predominantly in the spleen and bone marrow of the recipients of KSL cells transduced with SIV-pGPIbα-hFVIII (Fig. 5B, lower panel).

The vector-specific WPRE expression was measured by real-time quantitative RT-PCR to quantitate the mRNA expression level in each organ. As expected from the RT-PCR results, the bone marrow and spleen are the major sites in mice transplanted with KSL cells transduced with the SIV vectors (Fig. 5B). In accordance with the data on hFVIII transcription, hFVIII molecules were immunohistochemically detected in the bone marrow and spleen of both types of transduced mice (Fig. 6). It should be noted that cells expressing GPIbα concurrently expressed hFVIII in bone marrow obtained from mice transduced with SIV-pGPIbα-hFVIII (Fig. 6A).

Finally, the present inventors assessed whether the platelet-specific gene transduction using SIV-pGPIbα-hFVIII corrected the phenotype of FVIII-deficient hemophilia A mice. The FVIII antigen level of plasma with or without platelet activity was measured in FVIII-deficient transplanted mice 30 or 60 days after transplantation. The present inventors detected FVIII activity in the transplanted mice. Therein, 1% to 2% correction was noted in the plasma of mice transplanted with SIV-pGPIbα-hFVIII-transduced KSL cells (Fig. 7A). When platelets were stimulated with collagen and PMA, the plasma FVIII level increased to 2% to 3.5% (Fig. 7A). The mortality rate after cutting the tail was significantly improved in transduced mice (Fig. 7B). In addition, the level of ectopically expressed hFVIII was not reduced. Furthermore, inhibitors against hFVIII were not detected in mice transplanted with SIV-pGPIbα-hFVIII- transduced KSL cells 60 days after transplantation (data not shown).

In the present invention, *in vivo* gene transduction into platelets and megakaryocytes was attempted using SIV lentiviral vectors carrying a platelet-specific promoter. The production of transgene products using platelets has been attempted in transgenic mice (Kufrin, D., Eslin, D.E., Bdeir, K., Murciano, J.C., Kuo, A., Kowalska, M.A., Degen, J.L., Sachais, B.S., Cines, D.B., and Poncz, M. (2003) Antithrombotic thrombocytes: ectopic expression of urokinase-type plasminogen activator in platelets. Blood 102, 926-933; Yarovoi, H.V., Kufrin, D., Eslin, D.E., Thornton, M.A., Haberichter, S.L., Shi, Q., Zhu, H., Camire, R., Fakharzadeh, S.S., Kowalska, M. A., Wilcox, D.A., Sachais, B. S., Montgomery, R.R., and Poncz, M. (2003) Factor VIII ectopically expressed in platelets: efficacy in hemophilia A treatment. Blood 102, 4006-4013). However, the techniques of transgenic animal are not applicable to the therapy. In the inventors' system, the transduction of hematopoietic stem cells carrying the SIV lentiviral vector caused about 20% of platelets to express the transgene, and also enabled the phenotype of hemophilia A mice to be corrected. The present invention revealed for the first time that transduction of blood coagulation factor genes into platelets can correct the phenotype of hemorrhagic disorders.

The life of a megakaryocyte is limited to about 10 to 21 days (Wilcox, D.A., and White II, G.C. (2003) Gene therapy for platelet disorders: studies with Glanzmann's thrombasthenia. J. Thromb. Haemost. 1, 2300-2311). For this reason, hematopoietic stem cells are more practical as a target of transduction than megakaryocytes to establish long-term expression of target proteins in platelets. Since lentivirus can infect certain types of resting cells, there has been much interest in the application of lentiviral vectors to the transduction of hematopoietic cells.

Then, lentiviral vectors were shown to be able to efficiently transduce hematopoietic stem cells (Woods, N.B., Ooka, A., and Karlsson, S. (2002) Development of gene therapy for hematopoietic stem cells using lentiviral vectors. Leukemia 16, 563-569). Based on its intrinsic safety, the present inventors used the SIV lentiviral system to efficiently transduce platelet-targeting genes. The SIV lentiviral system derives from SIVagmTYO1, and is nonpathogenic to its natural host and to experimentally-infected Asian macaques (Nakajima, T., Nakamaru, K., Ido, E., Terao, K., Hayami, M., and Hasegawa, M. (2000) Development of novel simian immunodeficiency virus vectors carrying a dual gene expression system. Hum. Gene. Ther. 11, 1863-1874). Replication-competent virus particles are not detected in vector-infected cells, and the risk of developing replication-competent lentivirus particles in HIV carrier patients is significantly lower than with HIV-based vectors. Thus, SIV vectors are advantageous regarding the problem of safety and clinical applications of gene therapy.

Most reported studies have used the GPIIb promoter for megakaryocyte- and platelet-specific gene transduction. The present inventors used the GPIbα promoter as a platelet-specific promoter in this study, because the promoter activity of GPIbα is more potent than the promoter activity of UT-7/TPO- and CD34⁺-derived megakaryocytes. Another reason why the present inventors selected this platelet-specific promoter is that it functions at a late stage of megakaryopoiesis. The GPIIb gene is expressed in platelets and megakaryocytes; however, it is an early-stage gene in megakaryopoiesis (Lepage, A., Leboeuf, M., Cazenave, J.P., de la Salle, C., Lanza, F., and Uzan, G. (2000) The IIb 3 integrin and GPIb-V-IX complex identify distinct stages in the maturation of CD34+ cord blood cells to megakaryocytes. Blood 96, 4169-4177).

In conditional knockout mice in which the thymidine kinase gene is promoted by the GPIIb promoter, the administration of ganciclovir resulted in a dramatic reduction in the total number of platelets (Tropel, P., Roullot, V., Vernet, M., Poujol, C., Pointu, H., Nurden, P., Marguerie, G., and Tronik-Le Roux, D. (1997) A 2.7-kb portion of the 5' flanking region of the murine glycoprotein IIb gene is transcriptionally active in primitive hematopoietic progenitor cells. Blood 90, 2995-3004). In the bone marrow, erythroid and myeloid progenitors were also affected. This suggested the presence of GPIIb in progenitor cells (Tropel, P., Roullot, V., Vernet, M., Poujol, C., Pointu, H., Nurden, P., Marguerie, G., and Tronik-Le Roux, D. (1997) A 2.7-kb portion of the 5' flanking region of the murine glycoprotein IIb gene is transcriptionally active in primitive hematopoietic progenitor cells. Blood 90, 2995-3004).

Indeed, 18% of human CD34⁺ hematopoietic stem cells already express GPIIb, and therefore the appearance of GPIb was markedly delayed as compared to that of GPIIb. This indicates that GPIb is a later-stage marker in megakaryocytic maturation. Thus, platelets using the GPIbα promoter were expected to enable specific and limited expression of gene products than when the GPIIb promoter is used.

Another important experimental result herein showed that the expression of the eGFP gene promoted by the CMV promoter was significantly reduced in platelets, despite the high efficiency of CD45⁺ cell transduction *in vivo.* In general, reduction in the expression of a transgene resulting from a shortened protein half-life was even more significant in terminally differentiated blood cells (Wahlers, A., Schwieger, M., Li, Z., Meier-Tackmann, D., Lindemann, C., Eckert, H.G., von Laer, D., and Baum, C. (2001) Influence of multiplicity of infection and protein stability on retroviral vector-mediated gene expression in hematopoietic cells. Gene Ther. 8, 477-486). This may be mediated by the down-regulation of the transgene during differentiation. Stability of the encoded protein is associated with the expression of the transgene at least to the same extent as with the selection of the promoter or cis-elements influencing RNA processing in differentiated cells (Wahlers, A., Schwieger, M., Li, Z., Meier-Tackmann, D., Lindemann, C., Eckert, H.G., von Laer, D., and Baum, C. (2001) Influence of multiplicity of infection and protein stability on retroviral vector-mediated gene expression in hematopoietic cells. Gene Ther. 8, 477-486). In this context, use of the GPIbα promoter, which promotes the late-stage megakaryocyte differentiation, can be important for gene transduction into terminally differentiated anucleate platelets.

The inventors' strategy of platelet-dependent gene transduction has potential for not only inherited platelet disorders (Glanzmann's thrombasthenia and Bernard-Soulier syndrome) but also other hemorrhagic disorders. Hemophilia A is an X chromosome-linked bleeding disorder caused by defects in the *FVIII* gene and affecting 1 in 5000 males (Hoyer, L. W. (1994) Hemophilia A. N. Engl. J. Med. 330, 38-47). Hemophilia is considered suitable for gene therapy because the disease is caused by a single gene abnormality and therapeutic coagulation factor levels can vary over a broad range (5 to 100%) (Hoyer, L. W. (1994) Hemophilia A. N. Engl. J. Med. 330, 38-47).

Sustained therapeutic expression of FVIII has been achieved in preclinical studies using a wide range of gene transfer technologies targeted at different tissues (Lozier, J. (2004) Gene therapy of the hemophilias. Semin. Hematol. 41, 287-296). However, the emergence of neutralizing antibodies often limits the clinical applications (High, K. (2005) Gene transfer for hemophilia: can therapeutic efficacy in large animals be safely translated to patients? J. Thromb. Haemost. 3, 1682-1691), and the targeting of hematopoietic stem cells is not an exception. Transduction of the lentivirus FVIII gene into hematopoietic stem cells enables the production of therapeutic levels of FVIII (Kikuchi, J., Mimuro, J., Ogata, K., Tabata, T., Ueda, Y, Ishiwata, A., Kimura, K., Takano, K., Madoiwa, S., Mizukami, H., Hanazono, Y., Kume, A., Hasegawa, M., Ozawa, K., and Sakata, Y. (2004) Sustained transgene expression by human cord blood derived CD34+ cells transduced with simian immunodeficiency virus agmTYO1-based vectors carrying the human coagulation factor VIII gene in NOD/SCID mice. J. Gene. Med. 6, 1049-1060, Kootstra, N.A., Matsumura, R., and Verma, I.M. (2003) Efficient production of human FVIII in hemophilic mice using lentiviral vectors. Mol. Ther. 7, 623-631). However, previous methods have induced neutralizing antibodies against FVIII and as a result reduced the level of FVIII activity (Kootstra, N.A., Matsumura, R., and Verma, I.M. (2003) Efficient production of human FVIII in hemophilic mice using lentiviral vectors. Mol. Ther. 7, 623-631).

Gene therapy for hemophilia A using platelets has advantages in therapeutic applications, because the use of the platelet-specific system can prevent induction of inhibitors (neutralizing antibodies) by preventing the expression of FVIII in antigen-presenting cells. Furthermore, 10 to 30% of hemophilia A patients carry inhibitors resulting from administration of blood coagulation factors. Therefore, there is a risk that the coagulation effect is reduced, leading to severe bleeding. The platelet-dependent gene therapy of hemophilia A is also very useful in treating such patients carrying the inhibitors, because the blood coagulation factors stored in platelets are protected from the inhibitors in the bloodstream. Then, these can be released at the sites of thrombus formation such that blood coagulation is activated.

There is a report on therapeutic expression of GPIIb/IIIa in GPIIIa-deficient mice using an HIV-lentivirus vector carrying a GPIIIa cDNA under the control of the GPIIb promoter (Fang, J., Hodivala-Dilke, K., Johnson, B.D., Du, L.M., Hynes, R.O., White II, G.C., Wilcox, D.A. (2005) Therapeutic expression of the platelet-specific integrin, IIb 3, in a murine model for Glanzmann thrombasthenia. Blood (in press)). The study used a heterogeneous population of bone marrow cells as a source for stem cell transplantation and gene transduction.

The present inventors demonstrated efficient transduction into murine KSL hematopoietic cells by an SIV vector carrying the GPIbα promoter and phenotypic correction of hemophilia A mice. Initial-stage KSL cells are a nearly homogeneous population, and a single KSL cell can frequently provide long-term multilineage engraftment in lethally irradiated mice (Nakauchi, H., Sudo, K., and Ema, H. (2001) Quantitative assessment of the stem cell self-renewal capacity. Ann. N. Y. Acad. Sci. 938, 18-24). Targeting of primitive hematopoietic stem cells is thought to be a safer approach, because the number of transduced cells required for reconstitution is much lower than the number required when using a heterogeneous bone marrow population.

The development of leukemia in two children with combined immunodeficiency disease who were transplanted with retroviral vector-transduced bone marrow cells caused renewed concern regarding the risk of proviral sequences being integrated into chromosomal DNA (Hacein-Bey-Abina, S., von Kalle, C., Schmidt, M., Le Deist, F., Wulffraat, N., McIntyre, E., Radford, I., Villeval, J.L., Fraser, C.C., Cavazzana-Calvo, M., and Fischer, A. (2003) A serious adverse event after successful gene therapy for X-linked severe combined immunodeficiency. N. Engl. J. Med. 348, 255-256). A theoretical approach to reduce the risk of insertional mutagenesis would be the use of a transduction protocol that minimizes the total number of genetically modified cells (Mostoslavsky, G., Kotton, D.N., Fabian, A.J., Gray, J.T., Lee, J.S., and Mulligan, R.C. (2005) Efficiency of transduction of highly purified murine hematopoietic stem cells by lentiviral and oncoretroviral vectors under conditions of minimal in vitro manipulation. Mol. Ther. 11, 932-940). In this aspect, the present inventors' procedure using KSL cells transduced with the SIV lentiviral system is a practical approach for platelet-specific gene modification in clinical applications.

### Industrial Applicability

The present invention is useful in treating blood coagulation abnormalities caused by either quantitative abnormality of a blood coagulation factor or abnormality in the activity of a blood coagulation factor. More specifically, blood coagulation abnormalities caused by a reduction in the expression of a blood coagulation factor or in the activity of a blood coagulation factor can be treated based on the present invention. Diseases that can be treated by the present invention include the blood coagulation abnormality disorders shown below. Names of blood coagulation factors that are useful in treating each disease are shown in parenthesis. Specifically, each of the following blood coagulation abnormality disorders can be treated by applying the blood coagulation factor-encoding genes shown below to the present invention.
Hemophilia A (Factor VIII and Factor VIIa)
Hemophilia B (Factor IX)
Von Willebrand's disease (von Willebrand factor)
Factor I deficiency, afibrinogenemia, and hypofibrinogenemia (Factor I)
Factor II deficiency (prothrombin)
Factor V deficiency and parahemophilia (Factor V)
Factor VII deficiency (Factor VII)
Factor X deficiency (Factor X)
Factor XI deficiency (Factor XI)
Factor XIII deficiency (Factor XIII)

Of these blood coagulation abnormality disorders, hemophilia A and hemophilia B are significant diseases because they may involve a severe bleeding tendency and the patient number is large. By applying the present invention to hemophilia, high therapeutic effects can be achieved with a small number of treatments. Therefore, the present invention can actualize therapeutic methods that place little constraint on the daily life of hemophilia patients.

## Claims

1. An agent for treating a blood coagulation abnormality, which comprises as an active ingredient a lentiviral vector comprising a promoter specific to megakaryocytes and/or platelets which are derivatives thereof, and a polynucleotide encoding a blood coagulation factor operably linked to the promoter.

2. The therapeutic agent of claim 1, wherein the promoter is a GPIb promoter or a variant thereof.

3. The therapeutic agent of claim 2, wherein the polynucleotide encoding a blood coagulation factor is linked to the promoter via 5' UTR.

4. The therapeutic agent of claim 1, wherein the blood coagulation abnormality is hemophilia A and the blood coagulation factor is Factor VIII or a mutant thereof.

5. The therapeutic agent of claim 1, wherein the blood coagulation abnormality is hemophilia B and the blood coagulation factor is Factor IX or a mutant thereof.

6. The therapeutic agent of claim 1, wherein the blood coagulation abnormality is either hemophilia A or hemophilia B and the blood coagulation factor is Factor VII or a mutant thereof.

7. The therapeutic agent of claim 1, wherein the lentiviral vector is a simian immunodeficiency virus vector.

8. The therapeutic agent of claim 1, wherein the lentiviral vector is any one selected from the group consisting of equine infectious anemia virus vector, human immunodeficiency virus 1 vector, human immunodeficiency virus 2 vector, feline immunodeficiency virus vector, bovine febrile disease virus vector, and caprine arthritis encephalitis virus vector.

9. A hematopoietic stem cell which has been infected with a lentiviral vector comprising a promoter specific to megakaryocytes and/or platelets which are derivatives thereof, and a polynucleotide encoding a blood coagulation factor operably linked to the promoter.

10. A megakaryocyte and/or platelet which is a derivative thereof, infected with a lentiviral vector comprising a promoter specific to megakaryocytes and/or platelets which are derivatives thereof, and a polynucleotide encoding a blood coagulation factor operably linked to the promoter.

11. A method for producing either or both of a megakaryocyte and a platelet in which a blood coagulation factor is accumulated, wherein the method comprises the steps of: infecting a hematopoietic stem cell with a lentiviral vector comprising a promoter specific to megakaryocytes and/or platelets which are derivatives thereof, and a polynucleotide encoding a blood coagulation factor operably linked to the promoter; and culturing the hematopoietic stem cell infected with the lentiviral vector until it differentiates into a group of cells comprising either or both of a megakaryocyte and a platelet which is a derivative thereof.

12. A method for treating a blood coagulation abnormality, wherein the method comprises the steps of:
(1) infecting a hematopoietic stem cell with a lentiviral vector comprising a promoter specific to megakaryocytes and/or platelets which are derivatives thereof, and a polynucleotide encoding a blood coagulation factor operably linked to the promoter; and
(2) administering the hematopoietic stem cell of step (1) to a patient with blood coagulation abnormality.

13. The method of claim 12, wherein the hematopoietic stem cell comprises a hematopoietic stem cell collected from a patient.

14. The method of claim 13, wherein the hematopoietic stem cell comprises either or both of a bone marrow stem cell and a peripheral blood hematopoietic stem cell.

15. The method of claim 12, wherein the hematopoietic stem cell of step (1) is cultured and then administered to the patient.

16. A kit for treating blood coagulation abnormality, wherein the kit comprises the following elements:
a: a lentiviral vector comprising a promoter specific to megakaryocytes and/or platelets which are derivatives thereof, and a polynucleotide encoding a blood coagulation factor operably linked to the promoter; and
b: a reagent for collecting a hematopoietic stem cell.

17. The kit of claim 16 for treating blood coagulation abnormality, wherein the kit additionally comprises the following element c:
c: a culture medium for inducing the differentiation of a hematopoietic stem cell into a megakaryocyte.

18. The kit of claim 17 for treating blood coagulation abnormality, wherein the culture medium for inducing the differentiation of a hematopoietic stem cell into a megakaryocyte comprises at least the following substances:
transferrin;
insulin;
stem cell factor;
thrombopoietin;
interleukin-6;
Flt-3 ligand; and
soluble interleukin-6 receptor.

19. Use of a lentiviral vector for producing an agent for treating blood coagulation abnormality, wherein the lentiviral vector comprises a promoter specific to megakaryocytes and/or platelets which are derivatives thereof, and a polynucleotide encoding a blood coagulation factor operably linked to the promoter.
